# EUROPEAN PATENT SPECIFICATION

(11) **EP 4 150 080 B1**
(45) Date of publication and mention of the grant of the patent: **28.10.2026**
(21) Application number: 21803056.7
(22) Date of filing: 13.05.2021
(51) Int. Cl.: C12N 15/11, C12Q 1/68, C40B 40/06

(54) **ON DEMAND SYNTHESIS OF POLYNUCLEOTIDE SEQUENCES**
AUF ANFRAGE SYNTHESE VON POLYNUKLEOTIDSEQUENZEN
SYNTHÈSE À LA DEMANDE DE SÉQUENCES POLYNUCLÉOTIDIQUES

(30) Priority: 15.05.2020 US 202063025696 P
(43) Date of publication of application: 22.03.2023
(73) Proprietor: Telesis Bio Inc., San Diego, CA 92121 (US)
(72) Inventor: KANNAN, Krishna, San Diego, CA 92121 (US); GILL, John E., San Diego, CA 92121 (US); GIBSON, Daniel G., San Diego, CA 92121 (US); FU, Lixia, San Diego, CA 92121 (US)
(74) Representative: Kilburn & Strode LLP
(86) International application number: PCT/US2021/032344
(87) International publication number: WO 2021/231799

(56) References cited:
- WO-A1-2005/111236
- WO-A1-2011/150168
- WO-A1-2017/096322
- WO-A1-2019/224544
- WO-A1-2019/241290
- WO-A2-2005/017153
- US-A1- 2004 146 901
- US-A1- 2016 215 316
- US-A1- 2017 267 998

## Description

### FIELD OF THE INVENTION

The invention relates to synthetic biology and the assembly of polynucleotide molecules from a library of oligonucleotide parts.

### BACKGROUND INFORMATION

The fields of synthetic biology and gene editing and therapeutics have a continuing and growing need for oligonucleotides of diverse and known sequences. Existing methods for synthesizing small oligonucleotides involve chemical synthesis via solid-phase, sequential coupling of nucleotides to generate the oligonucleotide of desired length and sequence. Oligonucleotides produced are then released from the solid phase, deprotected, and collected for assembly into larger oligonucleotides by other methods. While automated, these processes are subject to side reactions and base errors, thus limiting the length of the oligonucleotides produced. For applications requiring ultra-high sequence fidelity, these methods have additional limitations.

Enzymatic methods of synthesizing oligonucleotides also exist and involve the use of enzymes such as terminal deoxynucleotidyl transferase (TdT), a template-independent polymerase that catalyzes the incorporation of deoxyribonucleotides into the 3'-hydroxyl end of DNA templates. But the enzyme shows strong bias for specific nucleotide bases and does not reliably add nucleotides in the desired order and length.

There is a continuing need for methods of synthesizing oligonucleotides efficiently and with high fidelity so that the user can produce oligonucleotides of any desired length and sequence.

WO 2005/017153 A2 describes methods for synthesis of defined polynucleotides.

WO 2011/150168 A1 describes methods and devices for in situ nucleic acid synthesis.

### SUMMARY OF THE INVENTION

The present invention is as set out in the appended claims.

The invention provides methods of synthesizing a product DNA molecule having a desired sequence in accordance with the appended claims. The product DNA molecule may have a defined sequence. The methods involve annealing at least one long oligonucleotide and at least one short oligonucleotide to at least one anchor strand having a sequence at least partially complementary to the at least one long and at least one short oligonucleotide. After annealing, at least one long oligonucleotide bound to an anchor strand abuts at least one short oligonucleotide bound to the same anchor strand. The anchor strand has one or more non-standard nucleotides, and one or more degenerate nucleotides. The method involves ligating the abutting at least one long oligonucleotide and at least one short oligonucleotide to form a dsDNA molecule. The dsDNA molecule is contacted with one or more enzymes that degrade DNA comprising non-standard nucleotides and the product DNA molecule is thereby synthesized. The invention also provides methods of synthesizing DNA molecules by assembling oligonucleotide members of a library that contains less than 20,000 members that can be assembled into all possible DNA sequences in accordance with the appended claims.

In a first aspect the invention provides a method of synthesizing a product DNA molecule having a desired sequence in accordance with the appended claims. The method involves annealing at least one long oligonucleotide and at least one short oligonucleotide to at least one anchor strand(s) having a sequence at least partially complementary to the at least one long and at least one short oligonucleotide. After annealing, at least one long oligonucleotide bound to an anchor strand abuts at least one short oligonucleotide bound to the same anchor strand. The at least one anchor strand has one or more non-standard nucleotides, and one or more degenerate nucleotides. The method further involves ligating the abutting at least one long oligonucleotide and at least one short oligonucleotide to form a dsDNA molecule comprising non-standard nucleotides, and contacting the dsDNA molecule with one or more enzymes that degrade DNA comprising one or more non-standard nucleotides to thereby synthesize the product DNA molecule.

In one embodiment the annealing occurs within a binding set comprising two long oligonucleotides, two short oligonucleotides, and two anchor strands, and further comprising ligating the two short oligonucleotides to the two long oligonucleotides, wherein the long and short oligonucleotides further comprise variable nucleotides. The annealing can occur within a binding set comprising two long oligonucleotides, one short oligonucleotide, and at least one anchor strand, and wherein the short oligonucleotide is ligated to two long oligonucleotides, and the short oligonucleotide and two long oligonucleotides comprise variable nucleotides. In any embodiment the non-standard nucleotide can be deoxy-uridine. The dsDNA molecule can have the non-standard nucleotides on only one strand.

In any embodiment the method can also include a step of amplifying the product DNA molecule using a DNA amplification method. In any embodiment the method can include performing multiple cycles of PCR on the product DNA molecule. The product DNA molecule can have flanking sequences for annealing a primer used in the multiple cycles of PCR. In any embodiment the product DNA molecule can be 8-30 nucleotides in length. The ligation can be performed by a DNA ligase. In one embodiment the at least one long oligonucleotide is from 12-24 nucleotides in length, and the at least one short oligonucleotide is from 3-8 nucleotides in length. In another embodiment the at least one long oligonucleotide is from 12-24 nucleotides in length, and the at least one short oligonucleotide is from 3-8 nucleotides in length, and the at least one anchor strand is from 20-60 nucleotides in length.

In any embodiment the method can include a step of ligating in solution two DNA molecules having double-stranded ends by blunt end ligation and according to the desired sequence. In one embodiment after annealing, a first short oligonucleotide abuts a first long oligonucleotide, and a second short oligonucleotide abuts a second long oligonucleotide, and the method involves ligating the first short and long oligonucleotides, and ligating the second short and long oligonucleotides.

In another embodiment after annealing the at least one short oligonucleotide abuts a first long oligonucleotide and a second long oligonucleotide, and the method involves ligating the at least one short oligonucleotide to the first and second long oligonucleotides. In various embodiments the one or more enzymes that degrade DNA comprising non-standard nucleotides are one or more enzymes selected from uracil DNA glycosylase, Endonuclease VIII, and Exonuclease T.

In any embodiment each anchor strand has 4-6 variable and/or 4-6 degenerate nucleotides present in a single degenerate region. In any embodiment the long and short oligonucleotides each have 4-6 variable nucleotides. In one embodiment the two long oligonucleotides also have variable nucleotides that bind to the degenerate nucleotides on their respective at least partially complementary anchor strands during the annealing step. In one embodiment the long oligonucleotides, short oligonucleotides, and anchor strand each comprise 4-6 variable nucleotides.

In some embodiment the product DNA molecule can be single-stranded DNA or double-stranded DNA 8-30 nucleotides in length, not including the flanking sequences. In other embodiments the product DNA molecule is single-stranded DNA or double-stranded DNA 16-20 oligonucleotide in length.

In any embodiment the long oligonucleotides and short oligonucleotides can be provided to the method from an oligonucleotide library. In some embodiments the short oligonucleotides provided from the oligonucleotide library are 3-7 nucleotides in length. The oligonucleotide library can include long oligonucleotide members having all four possible nucleotides at each position of a variable nucleotide. The long oligonucleotides can have a variable region that anneals to the sequence of degenerate nucleotides on the one or more anchor strands.

In any embodiment of the method the short and long oligos comprise 4-6 variable nucleotides, the anchor strands comprise 4-6 degenerate nucleotides, the anchor strands comprise at least two deoxy-uridine monophosphate nucleotides, and the product DNA molecule is amplified by PCR.

In any embodiment the product DNA molecule can have an error rate of fewer than 1 in 25,000. In any embodiment the at least one long oligonucleotide, at least one short oligonucleotide, and at least one anchor strand can be selected from an oligonucleotide library having less than 20,000 members, which oligonucleotide library can contain oligonucleotides sufficient to synthesize all possible nucleotide sequences.

In any embodiment the methods can also involve a step of contacting the product DNA molecule with a Type IIS restriction endonuclease to generate a product DNA molecule having single-stranded overhangs. The Type IIS restriction endonuclease can be any, for example BsaI and/or BsmBI. In one embodiment the product DNA molecule encodes a non-genetic message. The product DNA molecule can have a sequence corresponding to bytes of information. In any embodiment the multiple cycles of PCR can include a first step of PCR using primers having non-standard nucleotides set two nucleotides back from the 3' end of the primer.

In one embodiment the product DNA molecule can encode a gRNA, and the flanking sequences can encode one or more transcriptional elements. In any embodiment the transcriptional elements can be any one or more of a promoter, a Cas9 handle, and a terminator.

In another aspect the invention provides a method of synthesizing a product DNA molecule in accordance with the appended claims. The method involves providing a library of oligonucleotides wherein the library of oligonucleotides has fewer than 20,000 oligonucleotide members, and the oligonucleotide members in the library can be assembled into all possible polynucleotide sequences; and assembling oligonucleotide members from the library to obtain the product DNA molecule. In one embodiment the library of oligonucleotides is present on a solid phase and the oligonucleotide members are present at defined physical locations within the library of oligonucleotides. The solid phase can be a DNA chip. The product DNA molecule can be a double-stranded DNA 8-30 base pairs in length. The product DNA molecule can be subjected to continuing methods to ligate the product DNA molecule with at least one additional DNA molecule to produce a larger product DNA molecule. In some embodiments the library has fewer than 16,000 oligonucleotide members. The larger product DNA molecule can be at least 1,000 bp in length. The product DNA molecule can have an error rate of fewer than 1 in 10,000.

Also described herein is a kit containing a library of 20,000 or less (fewer) oligonucleotide members, where the oligonucleotide members in the library can be assembled into every possible polynucleotide sequence. The oligonucleotide members of the kit can be isolated at defined locations and spatially separated. In one embodiment the library is present on a DNA chip.

Also described herein is an oligonucleotide library having less than 20,000 defined locations and having an oligonucleotide library member at each location. The oligonucleotide library members can be assembled into every possible polynucleotide sequence. In one embodiment polynucleotide sequence is less than 10 Mbp in length.

### DESCRIPTION OF THE DRAWINGS

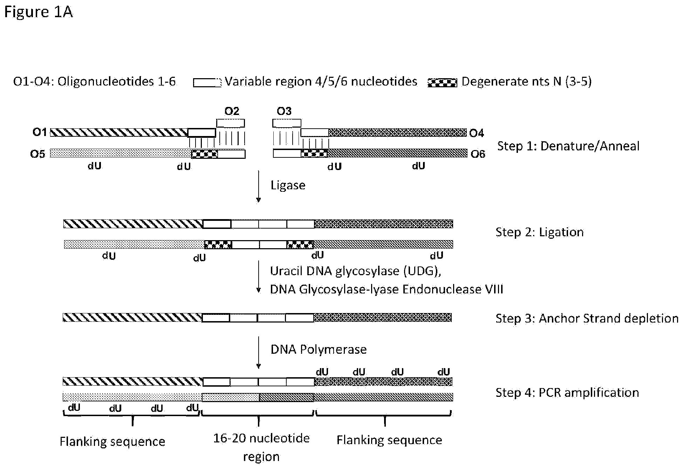

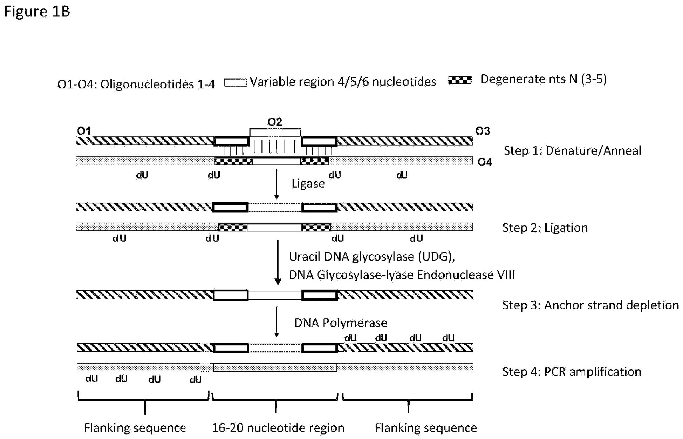

Figure 1A-1B; Figure 1A provides a schematic illustration of an embodiment of the invention termed Embodiment 1 (E1) using two short oligos and two long oligos and two anchor strands. O2 and O3 are short oligonucleotides composed of variable nucleotides. O2 binds to the variable region of O5 and O3 binds to the variable region of O6. Figure 1B provides a schematic illustration of an embodiment of the invention termed Embodiment 2 (E2) using one short oligo and two long oligos and one anchor strand. dU are depicted as part of the oligonucleotide sequence. O2 binds to the variable region of O4.

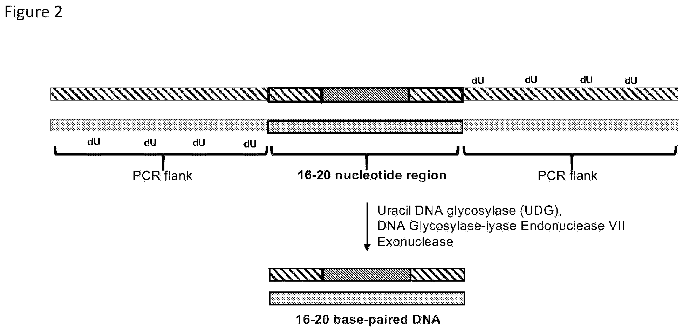

Figure 2 depicts an embodiment for the removal of flanks. Following the PCR reaction the dsDNA produced has 16-20 nucleotides of defined sequence flanked by universal primer sites for PCR. In this embodiment deoxy-uridine is the non-standard nucleotide, and degrading or digesting the anchor strand involves contact with uracil DNA glycosylase (UDG), and DNA Glycosylase-Endonuclease III and Exonuclease, resulting in an 8-30 nucleotide dsDNA of defined sequence. dU are depicted as part of the oligonucleotide sequence.

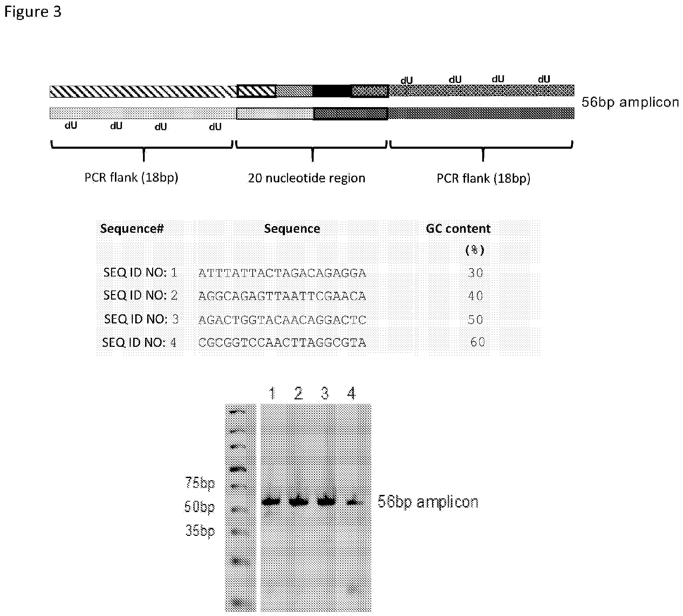

Figure 3 is a schematic illustration of the assembly of product DNA molecules of varying GC content. Four oligonucleotide sequences (SEQ ID NOs: 1-4) with a GC range of 30-60% were assembled according to the procedure of Example 1 and Figure 1A. 20 bp DNA products were assembled having 18 bp flanking sequences. dU are depicted as part of the oligonucleotide sequence.

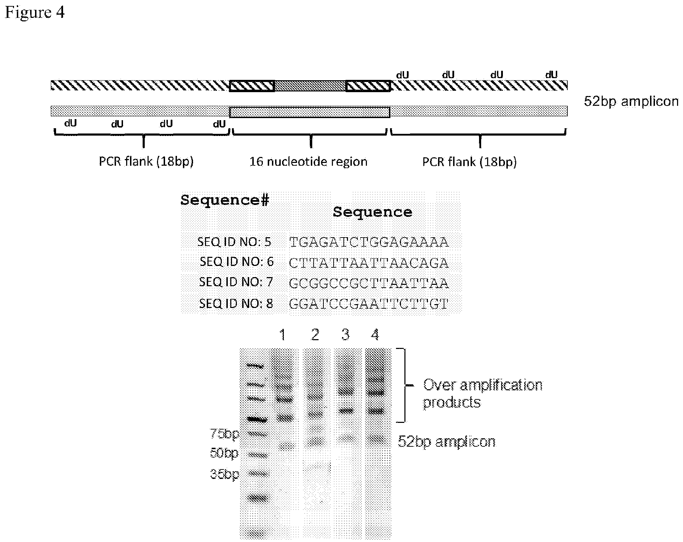

Figure 4 is a schematic illustration of the assembly of product DNA molecules of varying GC content. Four oligonucleotides sequences (SEQ ID NOs: 5-8) with a GC range of 30-60% were assembled according to the procedure of Example 2 and Figure 1B. 16 bp DNA products were assembled having 18 bp flanking sequences. dU are depicted as part of the oligonucleotide sequence.

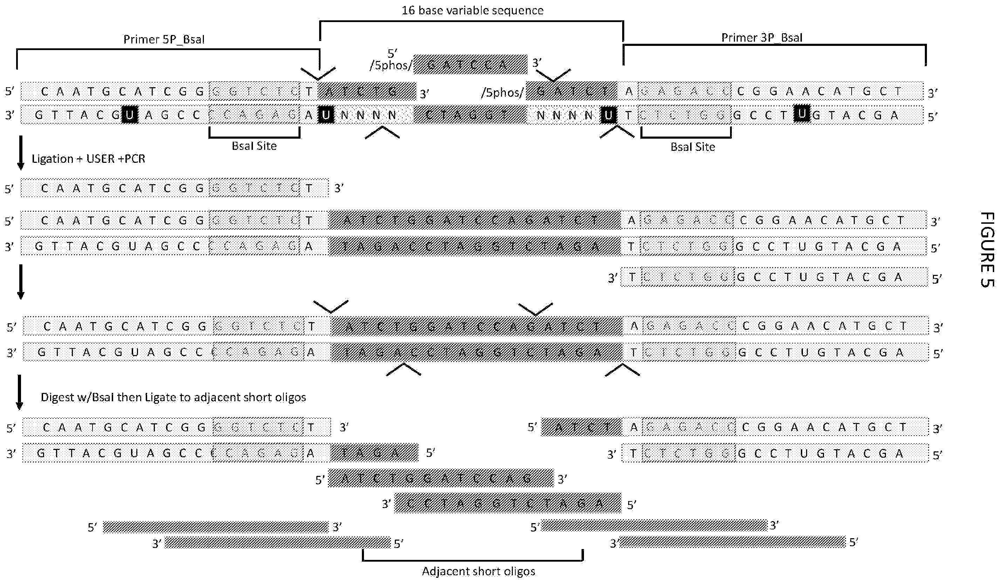

Figure 5 provides a schematic illustration of the use of restriction endonucleases in the invention to generate product DNA molecules having "sticky" ends. The restriction enzymes cut a defined number of nucleotides away from the recognition site. Figure 5 discloses SEQ ID NOS 51-57, 55-56, 54, 59, 58, 57 and 60-61.

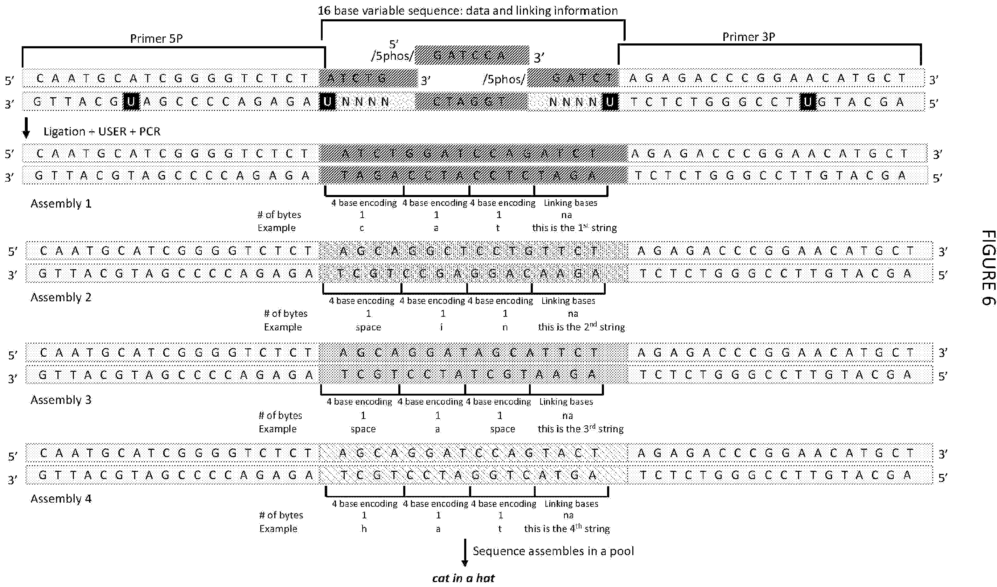

Figure 6 provides a schematic illustration of an embodiment of the invention for storing digital information in DNA. A 16 bp product DNA molecule is produced encoding four bytes of information. The examples shows how a non-genetic message (here "cat in a hat") can be encoded into DNA using the methods of the invention. Figure 6 discloses SEQ ID NOS 51-53, 55 and 62-68.

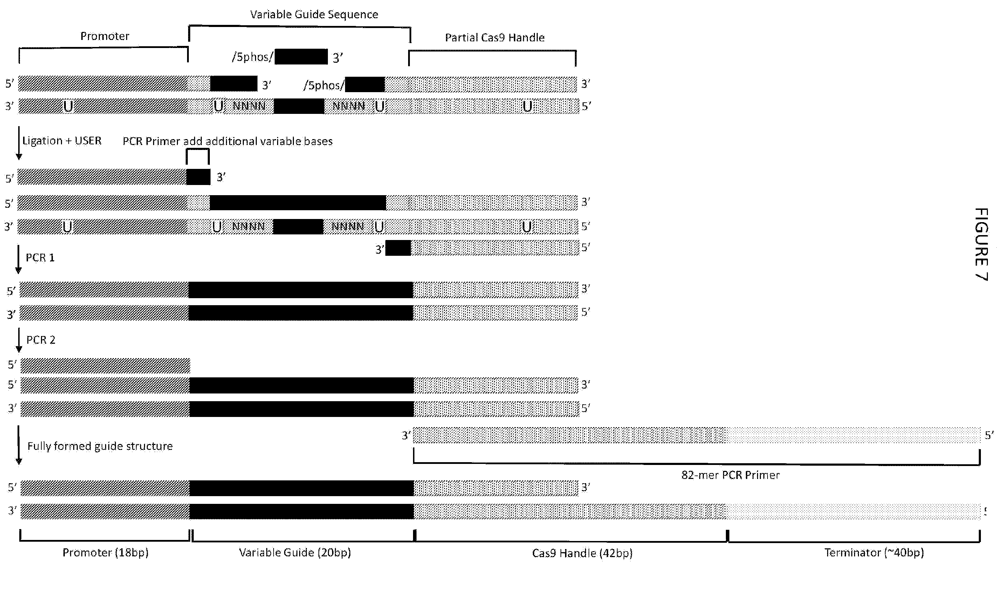

Figure 7 is a schematic illustration of an embodiment of the invention applied to synthesizing a 120 bp product DNA, which is an initial guide structure having the transcriptional elements of a promoter, a guide RNA, a Cas9 handle, and a terminator. In this embodiment the first cycle of PCR utilizes two primers having two variable bases on their 3' ends. This converts the otherwise 16 bp product DNA molecule into a 20 bp product. Later step(s) of PCR incorporate transcriptional elements.

### DETAILED DESCRIPTION OF THE INVENTION

The invention provides methods of assembling DNA molecules of any sequence with high fidelity using a library of oligonucleotides. The methods involve the use of an oligonucleotide library having DNA molecule members such that all possible DNA sequences can be assembled from the library using the methods. In one embodiment the library of oligonucleotides has less than 20,000 members.

Many efforts have been made towards achieving methods of assembling any possible DNA sequence from a library having a limited number of members. The present inventors discovered that any possible DNA sequence can be conveniently assembled using the materials and methods disclosed herein. The invention therefore enables creation of a library of less than 20,000 oligonucleotides, from which all possible oligonucleotide sequences can be assembled. The library of less than 20,000 oligonucleotides can be conveniently provided on a small device (e.g. a DNA chip), and devices and instrumentation provided to selectively assembly any DNA sequence using only the members of the oligonucleotide library.

In some embodiments the invention provides methods of synthesizing oligonucleotides of 8-30 nucleotides in length that can be used as building blocks for further assembly into larger oligonucleotides. The invention also provides a library of pre-made oligonucleotides, which can contain less than 20,000 members. The invention also provides methods of synthesizing DNA molecules by assembling oligonucleotides from the library. The members of the library can be assembled into all possible DNA sequences. Kits for conducting the methods are also provided. The invention does not rely on TdT (terminal deoxynucleotidyl transferase) enzyme, nor require its base-by-base synthesis of oligonucleotides.

### Oligo Library

In various embodiments the oligonucleotide members in the library can be DNA of various lengths. The library can have long and short oligonucleotide members. In various embodiments the library of oligonucleotides can have less than 50,000 members, or less than 40,000 members, or less than 30,000 members, or less than 20,000 members or less than 19,000 members or less than 18,000 members or less than 17,000 members or less than 16,000 members. The methods are able to synthesize all possible polynucleotide sequences using the oligonucleotide members in the library. In various embodiments the invention permits the assembly of over 4 billion (for a 16mer) and up to over 1 trillion (for a 20mer) polynucleotides beginning only with those oligonucleotides in the library. In various embodiments each oligonucleotide in the library can be used from 100 to 10,000 times in the synthesis of product DNA molecules. The product DNA molecule assembled can be of any size, for example it can be less than 1 Mbp or less than 5 Mbp or less than 10 Mbp or less than 12 Mbp, or less than 13 Mbp, or less than 14 Mbp, or less than 15 Mbp or 1-10 Mbp, or 1-12 Mbp, or 1-15 Mpb. The terms "oligo" and "oligonucleotide" are used interchangeably herein, and indicates a polymer of nucleotides of generally short length. "Polynucleotide" is a general term denoting a polymer of nucleotides of any length.

In other embodiments the methods can also be used with even smaller libraries to assemble a significant number of sequences that may be desired, for example the library can have fewer than 14,000 members or fewer than 12,000 members, or fewer than 10,000 members or fewer than 8,000 members, or fewer than 7,000 members, and be used to assemble a more limited and directed number of sequences in a defined category where such sequences are needed. Examples of a defined category can include a set of genes related to a specific biological function, or from a particular organism. Examples of defined categories can include (but are not limited to), genes or sequences related to transciption, regulation, RNA metabolism, translation, protein folding, protein export, RNA (rRNA, tRNA, small RNAs), ribosome biogenesis, rRNA modification, DNA replication, DNA repair, DNA topology, DNA metabolism, chromosome segregation, cell division, and tRNA modification.

In any embodiment the product DNA molecule synthesized in the method can be synthesized entirely or only from oligonucleotides from the oligonucleotide library. The oligo library can thus contain short oligonucleotides, long oligonucleotides, and anchor strands.

### Long and short oligonucleotides

In one embodiment the long and short oligonucleotides can be DNA of any length, but the long oligonucleotides are of greater length than the short oligonucleotides. For example, the short oligonucleotides can be up to 12 nucleotides in length, and the long oligonucleotides can be greater than 12 nucleotides in length. In various embodiments short oligonucleotides can have many lengths, examples include without limitation 4-6 nucleotides (nt), or 3-8 nt or 4-7 nt or 4-8 nt or 5-7 nt or 5-8 nt or 6-7 nt or 6-8 nt. In various embodiments long oligonucleotides can have a length of, for example and without limitation, 9-18 nt or 9-20 nt or 9-22 nt or 9-25 nt or 9-30 nt or 9-35 nt or 10-18 nt or 10-20 nt or 10-22 nt or 11-18 nt or 11-20 nt or 11-22 nt or 20-24 nt or 20-25 nt. Long and short oligonucleotides can be present in any combination or sub-combination of short and long oligonucleotide lengths provided herein.

In any embodiment the long oligonucleotides can be of greater length than the longest short oligonucleotide, for example the short oligonucleotides are 5-12 nucleotides in length, and the long oligonucleotides are at least 13 nucleotides in length. Thus, the long oligonucleotides can be long relative to the short oligonucleotides. In any embodiment the short and/or long oligonucleotides can have only nucleotides having no non-standard bases. In any embodiment the short and/or long oligonucleotides can have only nucleotides having standard bases, i.e. all nucleotides in the oligonucleotide have a base that is either A (adenine), T (thymine), C (cytosine), or G (guanine). Long oligonucleotides and/or anchor strands can have sequences for binding a primer, which can be used in PCR or another DNA amplification procedure.

Figure 1A depicts oligonucleotides and methods utilized in one embodiment of the invention. In this embodiment oligos 1-6 (01-06) all are depicted as having a variable region. O1 and O4 are long oligonucleotides and O2 and O3 are short oligonucleotides. O5 and O6 are anchor strands having non-standard nucleotides, indicated as deoxyuridine (dU). In Step 1 O1 and O2 anneal to O5, and O3 and O4 anneal to O6. This annealing can be simultaneous but in some embodiments does not have to be simultaneous. O2 can anneal to O5, and O3 can anneal to O6 and the long oligonucleotides can anneal subsequently. But oligos can anneal in any order. In this embodiment a long oligonucleotide and a short oligonucleotide each anneal so that they abut one another and are annealed to the same anchor strand. In any embodiment blunt end ligation can occur. A double-stranded DNA molecule is formed comprised of O1-O6, and therefore contains one strand of DNA having one or more non-standard nucleotides, which can all be on the same one of the two strands. In another step anchor strand depletion can occur. The anchor strand has one or more non-standard nucleotides, which contain a non-standard base. The dsDNA is contacted with one or more enzymes that degrade or digest DNA having one or more non-standard nucleotides. Degrading or digesting the DNA can involve excising one or more non-standard nucleotides (or the bases from those nucleotides), eroding the strand having the non-standard nucleotides and leaving a single-stranded DNA molecule, which can have no non-standard bases or only standard bases. The single-stranded DNA molecule can then optionally be amplified by PCR or another DNA amplification procedure, such as by contacting the single-stranded DNA molecule with primers and subjecting the mixture to PCR or another DNA amplification procedure. Primers used in PCR or other DNA amplification procedures can have one or more non-standard nucleotides (e.g. dU), in a similar or the same manner as anchor strands. O1, O4, O5 and O6 can have sequences for binding one or more primers suitable to accomplish the DNA amplification procedure, and which can be present as flanking sequences.

Figure 1B depicts another embodiment (Embodiment 2, E2). In this embodiment oligos 1-4 (01-04) all are depicted as having a variable region. O1 and O3 are long oligonucleotides and O2 is a short oligonucleotide. O4 is an anchor strand having non-standard nucleotides. In Step 1 O1, O2, and O3 anneal to O4. Again, this annealing may be, but does not have to be, simultaneous and O1, O2, and O3 can anneal in any order to O4. In this embodiment blunt end ligation does not occur and is not necessary. Thus, in some embodiments (including additional embodiments not depicted in Figure 1B) the methods do not involve blunt end ligation and blunt end ligation does not occur. A double-stranded DNA molecule is formed comprised of 01-04, and therefore contains one strand of DNA having one or more non-standard nucleotides from O4. In another step anchor strand depletion can occur. The anchor strand O4 has one or more non-standard nucleotides, which contain a non-standard base. The dsDNA is contacted with one or more enzymes that degrade or digest DNA having one or more non-standard nucleotides. Degrading or digesting the DNA can involve excising one or more non-standard nucleotides (or the bases from those nucleotides), eroding the strand having the non-standard nucleotides and leaving a single-stranded DNA molecule. The single-stranded DNA molecule can then be amplified by PCR or another DNA amplification procedure, such as by contacting the single-stranded DNA molecule with primers and subjecting the mixture to PCR or another DNA amplification procedure. O1, O3, and O4 in the embodiment can have sequences for binding one or more primers suitable to accomplish the DNA amplification procedure.

### Anchor Strands

In some embodiments the anchor strands can comprise one or more non-standard nucleotides. Non-standard nucleotides are nucleotides having a nucleobase other than C, G, A, or T. In some embodiments the non-standard nucleotide has uracil as a nucleobase (e.g. deoxy-uridine monophosphate). An AP site (also known as an abasic site) is a location in DNA that has neither a purine nor a pyrimidine base (nor a derivative of them), thus a nucleobase is missing; these are also a type of non-standard base. Other examples of non-standard nucleotides include, but are not limited to, dihydroxythymine, thymine glycol, an AP site, an apurinic site, an apyrimidinic site, 5-hydroxy-5-methylhydantoin, 5-hydroxy-hydantoin, and methyltartronylurea. Persons of ordinary skill in the art with reference to this disclosure will realize other non-standard nucleotides that will find use in the invention. As used herein deoxynucleotide can refer to the monophosphate, diphosphate, or triphosphate form. Thus, deoxyuridine can indicate any of dUMP, dUDP, or dUTP, which will be understood by the person of ordinary skill in the art through context of usage.

In some embodiments the anchor strands have one or more uracil-containing nucleotides as a non-standard nucleotide. The anchor stands also have one or more degenerate nucleotides, which can be present as a degenerate nucleotide region. In some embodiments the uracil-containing nucleotide contains deoxy-uracil, but in other embodiments the uracil-containing nucleotide can contain uracil glycol as a nucleobase. Uracil-containing non-standard nucleotides can also occur from cytosine deamination or incorporation of deoxyuridine phosphate residues. Such uracil-containing nucleotides can be incorporated into the anchor strand in some embodiments. In various embodiments an anchor strand can have 2 or 3 or 4 or 5 or 6 or more than 6 dU nucleotides in its sequence. In some embodiments the non-standard nucleotides can be spaced approximately evenly in the anchor strand. In various embodiments the anchor strand is an oligonucleotide that can have a uracil-containing nucleotide every 7 or every 8 or every 9 or every 10 or every 12 or every 15 nucleotides. While spaced non-standard nucleotides are sufficient, anchor strands can also have one or more consecutive non-standard nucleotides, in addition to or instead of spaced non-standard nucleotides. Anchor strands can be of any suitable length depending on the application, but in various embodiments the anchor strand can be 20-30 nucleotides in length, or 25-30 nucleotides in length, or can be from 25-40 or from 25-85 nucleotides or from 25-80 nucleotides or from 25-70 nucleotides, or from 25-65 nucleotides, or from 25-60 nucleotides in length. But in other embodiments the anchor strands can also be 18-65 or 18-85 or 20-65 or 20-85. In various embodiments of Embodiment 1 (E1) (Figure 1A) the anchor strands can be 20-35 nucleotides or 20-30 nt or 25-30 nt in length. In various embodiments of Embodiment 2 (E2) (Figure 1B) the anchor strands can be 30-40 nucleotides or 30-55 or 40-60 nucleotides in length. In any embodiment the anchor strand can be about the length of a long oligo and a short oligo combined, or 80-120% or 90-110% of the length of a long oligo and a short oligo combined. In any embodiment the anchor strand can be about the length of two long oligos and one short oligo combined, or 80-120% or 90-110% of the length of two long oligos and one short oligo combined.

Various embodiments using different length of short oligos, long oligos, and anchor strands can be utilized. In various embodiments of E1 or E2 (Figures 1A and 1B) the short oligonucleotides can be 4-6 nucleotides in length and the long oligonucleotides can be 14-22 nt in length. In other embodiments of E1 the short oligonucleotides can be 5-6 nucleotides in length and the long oligonucleotides can be 14-22 nt in length; anchor strands can be 20-30 nt in length. In another embodiment the short oligonucleotides are 4-6 or 5-6 nucleotides in length and the long oligonucleotides are 16-20 nt in length; anchor strands can be 20-35 nt in length. In some embodiments of E2 the short oligonucleotides are 4-6 or 5-6 nucleotides in length and the long oligonucleotides are 16-20 nt in length; anchor strands can be 40-60 nt in length. Persons of ordinary skill with resort to this disclosure will realize that the length of short oligos, long oligos, and anchor strands can be adjusted depending on the specific application contemplated.

In any embodiment a DNA sequence containing one or more non-standard nucleotide(s) is a substrate for one or more enzyme(s) that degrade DNA containing non-standard nucleotide(s). When the non-standard nucleotide is a uracil-containing nucleotide it can be a substrate for the one or more enzymes that degrade DNA containing uracil-containing nucleotide(s). In one embodiment the enzyme is uracil DNA glycosylase (UDG), which catalyzes the hydrolysis of the N-glycosidic bond from deoxyuridine to release uracil. The uracil-containing nucleotide can also be a substrate for endonuclease VIII, which has DNA glycosylase activity and AP lyase activity - thus, the endonuclease VIII can cleave uracil from the nucleotide. In one embodiment USER^{®} II can be utilized to excise uracil (New England Biolabs, Ipswich, MA). USER^{®} II has uracil DNA glycosylase activity (which excises a uracil base) and Endonuclease III having lyase activity (which breaks the phosphodiester backbone at the 3' and 5' sides of the abasic site), thus promoting the degradation of DNA. The uracil-containing nucleotide can also be a substrate for Endonuclease VIII, which has N-glycosylase activity and an AP-lyase activity. Endonuclease VIII can also be used when the non-standard nucleotide is a damaged pyrimidine, which it will cleave to leave an apurinic site. Endonuclease VIII can be used in various embodiments where the non-standard base is any of urea, 5,6-dihydroxythymine, thymine glycol, 5-hydroxy-5-methylhydantoin, uracil glycol, 6-hydroxy-5,6-dihydrothymine, or methyl tartronylurea. Endonuclease III can also recognize and remove nucleotides having these non-standard bases. An exonuclease (e.g. exonuclease T, exonuclease I, and others) can be used to remove single-stranded overhangs. Persons of ordinary skill with resort to this disclosure will realize many other non-standard bases and manners of degrading DNA containing them, which will find application in the present invention.

Anchor strands are at least partially complementary to at least one short oligonucleotide and at least one long oligonucleotide. In some embodiments the anchor strands are fully complementary to at least one short oligonucleotide and/or at least one long oligonucleotide. Anchor strands can, optionally, have 4, 5, 6, 7, 8, or 4-6 or 4-7 or 4-8 or more than 8 degenerate nucleotides, which can be present as a consecutive sequence or dispersed singly or in groups in the molecule. A "region" of degenerate nucleotides is two or more consecutive degenerate nucleotide locations.

### Methods

The methods of the invention synthesize a product DNA molecule having a desired sequence, which can be a pre-determined sequence, i.e. one decided by the user prior to beginning the method. In various embodiments the product DNA molecule of desired sequence can be 16-20 nucleotides in length, or 6-12 or 6-16 or 6-20 or 6-24 or 12-24 nucleotides in length, or 12-25 or 12-30 or 12-40 or 12-60 or 20-25 or 20-30 or 20-40 or 20-60 or 20-80 or 60-120 or 60-180 nucleotides in length. The product DNA molecule can be optionally assembled having flanking sequences, useful for continuing procedures (e.g. PCR or other DNA amplification). Flanking sequences can be of any length appropriate for the continuing procedures contemplated, for example about 12 nucleotides, or about 18 nucleotides, or about 18-22 nucleotides or 18-30 nucleotides or 18-60 nucleotides. The methods involve annealing at least one long oligonucleotide and at least one short oligonucleotide to at least one anchor strand(s) having a sequence at least partially complementary to the at least one long and at least one short oligonucleotide. In any embodiment the long and short oligo(s) can be completely complementary to a portion of the anchor strand(s). The annealing can be a partial annealing where only at least 50% or at least 70% or at least 90% of the nucleotides in the short and/or long oligonucleotides are annealed to their complementary nucleotides on the at least one anchor strand, or can be a complete annealing where all of the nucleotides in the short oligonucleotide and/or long oligonucleotide are annealed to the complementary sequence on the at least one anchor strand. After annealing the at least one long oligonucleotide bound to an anchor strand abuts at least one short oligonucleotide bound to the same anchor strand. In some embodiments the at least one long oligonucleotide can abut two short oligonucleotides bound to the anchor strand, and/or the at least one short oligo can abut two long oligos bound to the anchor strand. The at least one long oligo and the at least one short oligo can be annealed or bound to the same anchor strand. In one embodiment at least one short oligo is abutted by two long oligos and is annealed between the two long oligos, where the short and long oligos are annealed to an anchor strand. In any embodiment the terms binding to the same anchor strand, or any two oligos in the methods being "bound," can mean direct binding, or can refer to two polynucleotides that are annealed, or to polynucleotides bound by hydrogen bonding. The hydrogen bonding can be of a strength sufficient to hold the two polynucleotides together in the steps of the methods disclosed herein (e.g. at 25 °C for at least 2 minutes).

The at least one long and at least one short oligonucleotide that abut one another and are annealed to the same anchor strand can be ligated to form a double-stranded DNA molecule (dsDNA). The ligation can be performed by a ligase, for example T4 DNA ligase, but any suitable DNA ligase can be utilized in the invention (e.g. Taq ligase). The dsDNA molecule can then be contacted with one or more enzymes that degrade DNA comprising uracil-containing nucleotides to synthesize the product DNA molecule.

The methods therefore allow the production of a product DNA molecule of 8-30 or 8-40 or 8-50 or 8-60 or 8-70 nucleotides (not counting flanking sequences) in length without the need for a conventional oligonucleotide synthesizer, which typically relies on chemical synthesis (e.g. phosphoramidite chemistry). Instead, the methods rely on enzymatic synthesis, and therefore such oligonucleotides of 8-30 or 8-40 or 8-50 or 8-60 or 8-70 or 8-80 or 8-100 nucleotides in length after removal of flanking sequences can therefore be produced on demand. In other embodiments the methods can produce a product DNA molecule of 6-30 or 6-40 or 6-70 or 6-90 or 6-120 nucleotides, not counting flanking sequences.

The methods can also involve performing multiple cycles of PCR or another DNA amplification procedure on the product DNA molecule. PCR is known to those of ordinary skill in the art and involves the use of primers. In any embodiment primers used in PCR or other DNA amplification procedures can contain one or more dU nucleotides, or other non-standard nucleotides. In some embodiments the primers can have two or three or four or more than four dU nucleotides, or other non-standard nucleotides.

In any embodiment the methods are performed without the use of restriction enzymes. In any embodiment the methods also can be performed without cloning or the need for cloning. In any embodiment the methods can be performed entirely in vitro. In any embodiment the methods can be performed without the use of live cells in any step of the method. In any embodiment the methods can produce a scarless product DNA molecule. By scarless DNA is meant DNA that does not have any nucleotide(s) introduced by or from the process of synthesizing the DNA (e.g. residue nucleotides from a linker or flanking sequence). In any embodiment the methods can produce a product DNA molecule that is barcode free, or free of a nucleotide sequence placed for identification purposes. A barcode can be a sequence that is not otherwise needed but has a particular sequence and is used to identify a sequence of DNA. In various examples and embodiments a barcode sequence is 6-8 nucleotides in length, or 4-10 nucleotides in length. In any embodiment the methods can be performed without any part of any oligonucleotide used in the method being immobilized, i.e. bound to a solid phase or solid support (e.g. a bead, DNA chip, microfluidic surface, etc). In any embodiment the oligonucleotides can be annealed in solution, and can be ligated in solution, i.e. without any oligonucleotide in the step or method being bound or partially bound to a solid phase or solid support. In any embodiment the methods of the invention can synthesize the product DNA molecule according to the methods described herein without the use of and without performing chemical assembly techniques (e.g. phosphoramidite chemistry). In any embodiment the methods of the invention can assemble the product DNA molecule using only enzymatic assembly of oligonucleotides. In any embodiment the methods can be performed by drawing the short oligonucleotides, long oligonucleotides, and anchor oligonucleotides from a library comprising less than 20,000 members, or from any library described herein. In any embodiment the at least one long oligonucleotide, at least one short oligonucleotide, and at least one anchor strand can be selected from an oligonucleotide library having less than 20,000 members, or from any oligo library described herein.

Figure 1A illustrates an embodiment of the invention involving blunt end ligation. In some embodiments a short oligonucleotide can anneal to an anchor strand to form a DNA molecule that has a blunt end, and optionally is partially single-stranded and partially double-stranded. For example with reference to Figure 1A, O2 can anneal to O5 to form such a DNA molecule being partially single-stranded and partially double-stranded and having a blunt end; correspondingly, O3 can anneal to O6 to form another such molecule. In any embodiment two blunt-end double-stranded DNA molecules (whether partially or fully double-stranded), for example O2-O5 and O3-O6 (or 01-02-05 and O3-O4-O6) depicted in Figure 1A, can be joined in solution in a blunt end ligation by a DNA ligase to form a longer DNA molecule. Binding of long oligonucleotides O1 and O4 so that O1 and O2 abut one another, and O3 and O4 abut one another can also occur. Subsequent ligation of abutting oligonucleotides by contacting the oligos with a DNA ligase can form the DNA molecule. Thus, in some embodiments formation of the product DNA molecule can involve one or more steps of blunt-end ligation of two at least partially double-stranded DNA molecules. However, in other embodiments O1 and O2 can anneal with O5 to form abutting O1 and O2 (01-02-05), and O3 and O4 can anneal with O6 to form abutting O3 and O4 (O3-O4-O6), and the DNA molecules formed can be ligated in solution by blunt end ligation through the action of a DNA ligase to form the DNA molecule.

Thus, in one embodiment a long oligonucleotide and a short oligonucleotide can both be bound to the same anchor strand to form a blunt end DNA molecule, and can be optionally ligated together, and can then be joined by blunt end ligation to another long and short oligonucleotide that are both bound to a different anchor strand and also form a blunt end DNA molecule. In any embodiment the anchor strand can be longer than the short oligo and the long oligo and no longer than the combined lengths of both the short oligo and the long oligo. Note that the anchor strands have non-standard bases (dU in Figures 1A and 1B). The ligated dsDNA can be contacted with enzymes that degrade DNA having non-standard bases, e.g. UDG and Endonuclease III or DNA glycosylase-lyase Endonuclease VIII. When the anchor strand is depleted a single-stranded DNA molecule is left.

As illustrated in Figure 1B, in some embodiments a short oligonucleotide and two long oligonucleotides can anneal to the same anchor strand, and the short oligonucleotide can be annealed to the anchor strand in between and abutting the two long oligonucleotides. The two long oligos, short oligo, and anchor strand can be a binding set, and the binding set can be optionally partly single-stranded and partly double-stranded. With reference to Figure 1B, O1 and O3 are long oligonucleotides, O2 is a short oligonucleotide, and O4 is an anchor strand. O1-O3 can bind in any order to anchor strand O4. The two long oligos and one short oligo can be bound to the anchor strand O4 where O2 is in between and abutting O1 andO3. O2 can be ligated (e.g. by ligase) to O1 and O3 after the oligos have bound to anchor strand O4. O2 can also spontaneously ligate to O1 and O3.

The product DNA molecule can optionally be formed having flanking sequences to the 3' and 5' sides of the product DNA molecule. These flanking sequences in one embodiment can provide binding sites for primers in amplification procedures (e.g. by PCR). The flanking sequences can optionally also have non-standard nucleotides for removal later. Thus, the product DNA molecule will predominate and be synthesized in the method. In some embodiments the product DNA molecule will contain flanking sequences having the primer binding sites. In any embodiment the methods can include a step of removing flanking sequences after amplification to yield the product DNA molecule. Methods of removing flanking sequences are known in the art. One such method is found in US 2018/0163254 A1, published June 14, 2018. In some embodiments the flanking sequences can be utilized to add length to the product DNA molecule, or to surround the product DNA molecule with transcriptional elements or other beneficial sequences that will be utilized in the final product. For example, the flanking sequences can be set to provide a promoter in front of the product DNA molecule, and/or to provide a terminator. In one embodiment the product DNA molecule is a 16-20 bp gRNA sequence, and the flanking sequences can be set to provide a promoter in front of the gRNA sequence, and a Cas9 handle and terminator after it. Thus, in some embodiments the product DNA molecule can be expanded to encompass the flanking sequences as well, which can provide more utility than being only binding sites for primers.

Any of the methods disclosed herein can be performed in an automated method, for example by an automated instrument. An automated method is one where no human intervention is necessary after the method is initiated - the method goes to completion from that point without a human having to perform any action. The automated instrument can contain components for selecting oligonucleotide members from the oligo library. A DNA sequence to be assembled can be uploaded, recorded on, or stored on a non-transitory computer-readable medium. A non-transitory computer-readable medium can be programmed to execute automated steps when inserted into or otherwise in electronic communication with a processor attached to or comprised within the automated instrument. The automated steps can be those disclosed herein for performing a method disclosed herein. Thus, the invention also provides a non-transitory computer-readable medium that is programmed with the locations of each member of an oligonucleotide library described herein, where the oligonucleotide library is present on a suitable support structure for the oligo library. In one embodiment the non-transitory computer-readable medium is programmed with the locations of 14,000-20,000 or 15,000-20,000 or 16,000-20,000, or up to 20,000, or 14,000-25,000 oligonucleotide library members. The medium can also be programmed with instructions to combine 4-6 members of a binding set from the library and to assemble the members of the binding set into a product DNA molecule according to the methods described herein.

Also described herein are kits having an oligo library described herein located on a medium. The medium can be any suitable medium, for example one or more of a DNA chip, one or more bead(s), one or more of a 96 well plate, one or more of a 384 well plate, one or more microfluidic reaction support(s), one or more microtiter plate(s), one or more nanotiter plate(s), one or more picotiter plate(s), or other solid support or solid phase surface that can retain oligonucleotide members of the library. When more than one medium is utilized the media can be present in numbers sufficient to accommodate the oligo library. The medium containing the oligonucleotide library can contain members in any suitable volume, and examples include volumes of 1 nl up to 100 ul, or 10 nl up to 100 ul,. A DNA chip (or DNA microarray) is a solid surface having a collection of microscopic locations, to which oligonucleotides can be attached and/or stored.

The methods of the invention can synthesize a product DNA molecule with a very low error rate. In various embodiments the methods can produce any product DNA molecule described herein with error rates of less than 1 in 10,000 nucleotides, or less than 1 in 12,000 nucleotides, or less than 1 in 15,000 nucleotides, or less than 1 in 25,000 nucleotides, or less than 1 in 30,000 nucleotides, or less than 1 in 40,000 nucleotides, or less than 1 in 50,000 nucleotides.

In any embodiment the methods disclosed herein do not utilize or require the use of a vector in the methods.

### Binding Sets

A binding set is a group of oligonucleotides where each oligonucleotide in the set can bind to at least one other oligonucleotide in the set in a method of synthesizing a product DNA molecule as described herein. In one embodiment a binding set contains two long oligonucleotides, two short oligonucleotides, and two anchor strands (e.g. the binding set depicted in Figure 1A). In another embodiment a binding set can contain two long oligonucleotides, one short oligonucleotide, and at least one anchor strand (e.g. as depicted in Figure 1B). In other embodiments a binding set can contain a long oligo and a short oligo and an anchor strand having sequences complementary to both. Binding sets can be bound to other binding sets to form a larger DNA molecule or to form the product DNA molecule of desired sequence. However, persons of ordinary skill with resort to this disclosure will realize that other binding sets utilizing other combinations of oligonucleotides can be assembled and be effective for assembling the product DNA molecule according to the methods described herein, or variations of them. For example, a binding set could utilize more than two anchor strands, or more than two long oligonucleotides, or more than two short oligonucleotides. Additional binding sets that can be utilized in the methods are also described herein. Such alternative embodiments of binding sets can be annealed so that at least one long oligonucleotide and at least one short oligonucleotide anneal to an anchor strand, and the at last one long oligonucleotide abuts at least one short oligonucleotide bound to an anchor strand.

These are only exemplary binding sets that can find use in the methods and should not be considered limiting. In other embodiments a binding set can contain three or four or five or six short oligos and two or three or four long oligos. In any embodiment multiple anchor strands can be used. In only some examples three or four or five or six anchor strands can be utilized in a binding set and find use in the methods disclosed herein. In only some exemplary embodiments four short oligos can be applied with two long oligos and one or two anchor strands. Short oligos, long oligos, and anchor strands can therefore be applied in any combination, and the person of ordinary skill with resort to this disclosure will be able to arrive at multiple combinations of oligos and anchor strands that can be applied in the methods.

### Variable regions

In various embodiments short and/or long oligonucleotides, and anchor oligos can have one or more variable nucleotides. Any one or more of the oligos can have 3 or 4 or 5 or 6 or 7 or 3-7 or 4-5 or 4-6 or 4-7 variable nucleotides in one or more regions or portions of the oligonucleotide sequence having variable nucleotides. The one or more variable nucleotides can be present as one consecutive sequence to comprise a variable region, or the variable nucleotides can be separated singly or in groups of two or more consecutive nucleotides throughout the oligo sequence to comprise a variable region. Oligonucleotides having variable nucleotides can represent a different sequence for each possibility presented by the variable nucleotides. For example, O1 can have a variable region. When the variable region is five nucleotides, O1 can have 1024 possible nucleotide sequences, i.e. 4x4x4x4x4 equals 1024 variable sequences for O1. The same is true for O2-O6 as depicted in Figure 1A and for O1-O4 depicted in Figure 1B, which show embodiments where any of the oligonucleotides can have one or more variable nucleotides in one or more regions of the oligo sequence. For short oligonucleotides each nucleotide of the oligo can be variable, i.e. the entire short oligo can be one variable region. But some short oligos can also have variable nucleotides dispersed in the sequence, singly or in groups as explained above. Each variable sequence of an oligo can be located at a distinct location in the oligo library.

### Degenerate Nucleotides

One or more anchor strands used in the methods have one or more degenerate nucleotides. A degenerate nucleotide in an oligo of defined sequence is a nucleotide that can be any of A, C, T, or G. An oligo having degenerate nucleotides is a degenerate oligonucleotide. While similar to oligos having variable nucleotides, degenerate oligos are co-located at the same (degenerate oligonucleotide) location in an oligo library. Degenerate oligos can thus be present at a location as a group, with each degenerate oligo having a separate sequence due to the degenerate nucleotides. In some embodiments degenerate nucleotides on one oligo can anneal to a variable region on another oligo, such as is depicted in Figures 1A-B. In various embodiments any of the oligonucleotides in a binding set can have degenerate nucleotides, and any of the oligonucleotide can have variable nucleotides. In some embodiments at least two oligonucleotides in a binding set have degenerate nucleotides. With reference to Figure 1A O5 and O6 can have degenerate oligonucleotides, or O1 and O4, or O2 and O3, and not the other oligonucleotides in the binding set. With reference to Figure 1B, O4 can have degenerate nucleotides, or O1 and O3 can have degenerate nucleotides, or O2 can have degenerate nucleotides, and not the other oligonucleotides in the binding set.

The one or more anchor strands can have 3 or 4 or 5 or 6 or 7 or 8 or 3-5 or 3-6 or 3-7 or 3-8 or 4-5 or 4-6 or 4-7 or 4-8 degenerate nucleotides. The one or more degenerate nucleotides in an oligo can be present as one consecutive sequence to comprise a degenerate region, or the degenerate nucleotides can be separated singly or in groups of two or more consecutive degenerate nucleotides throughout the oligo (e.g. an anchor strand). Oligonucleotide members of the library having degenerate nucleotides have multiple possible sequences and can be grouped together and located at a single location in the library, and be considered as one oligonucleotide member of the library. Thus, an anchor oligo (or other oligo) having a degenerate region of, for example, five degenerate nucleotides can have 1024 possible sequences (4x4x4x4x4), but all 1024 sequences can be co-located at a single defined location in the library. A location in the oligo library containing the multiple sequences of degenerate oligonucleotides is termed a degenerate oligonucleotide location. Multiple degenerate oligonucleotides (each of slightly different sequence) can be co-located at a single location in the oligo library. While in some embodiments all possible sequences of a degenerate oligonucleotide are located at the same location (e.g. all 1024 possible sequences of a degenerate oligo having 5 degenerate nucleotides), in other embodiments multiple degenerate oligonucleotides can be located in groups of convenient numbers at multiple different locations in the oligo library.

In addition to a degenerate sequence, anchor oligonucleotides can also have one or more variable nucleotides, which can be located in a variable region of the oligo. When an anchor (or other) oligo has variable nucleotides, for example five variable nucleotides, there are 1024 possible variable sequences (4x4x4x4x4) of the oligo. When oligos have one or more variable nucleotides each variable sequence of the oligo can be located at its own defined location in the library. Thus, while oligos having one or more degenerate nucleotides are co-located at a single defined location in the library, oligos having variable nucleotides can each have their own defined location in the library, a separate location for each possible variable sequence. An oligonucleotide such as an anchor strand having one or more degenerate nucleotides can be co-located at a single location having all 1024 possible sequences of the anchor strand having degenerate nucleotides present at the single location. When an oligonucleotide having variable nucleotides also has degenerate nucleotides, the degenerate sequences can also be present at all 1024 locations of the oligos having variable nucleotides, and each oligo at a defined location having the degenerate sequences can have the same variable sequence at that location.

For illustration, consider oligo O5 in Figure 1A having a variable region of five nucleotides, and a degenerate region of five nucleotides. O5 can thus be present at variable locations L1...L1024 for O5, with each location having each of degenerate sequences D1...D1024 and a distinct variable sequence. Thus, degenerate sequences D1-D1024 can all be present at each of variable locations L1-L1024 for O5, each having a distinct variable sequence. Thus, at location L1 for O5 degenerate sequences D1-D1024 will all have variable sequence V1. At location L2 for O5, degenerate sequences D1-D1024 will all have variable sequence V2. At location L3 for O5 degenerate sequences D1-D1024 will all have variable sequence V3, and so on. Thus, degenerate sequences D1-D1024 for O5 are all present at locations L1-L1024 for O5, with each degenerate sequence having the variable sequence for the particular location. With respect to O1-O4 in Figure 1A, these oligos have only variable regions and no degenerate nucleotides. Thus, if each has a variable region of five nucleotides, 1024 possible sequences exist for each oligo (4x4x4x4x4), which can be present at 1024 distinct locations in the library (L1...L1024) for each oligo. Thus, a variable region on one oligo can anneal to a variable region and/or to a degenerate region on a complementary oligo.

### Oligonucleotide Library

The invention also provides methods of synthesizing a product DNA molecule involving providing a library of oligonucleotide members. The library of oligonucleotide members can have fewer than 20,000 oligonucleotide members, and the oligonucleotide members in the library can be assembled into all possible polynucleotide sequences. The method involves assembling oligonucleotide members from the library to obtain the product DNA molecule.

With reference to Figure 1A all oligonucleotides, O1-O6, are members in the library. O1, O2, O3, and O4 can each optionally have one or more variable regions. The oligonucleotide library can be one or more DNA chip, solid support, solid phase, bead, microfluidic surface, plate, etc, or other structure where oligonucleotides can be stored at defined locations and be available for retrieval and use in the methods. Thus, in some embodiments the library will contain a distinct location for each of the possible variable sequences of O1, O2, O3, and O4 (in embodiments where O1-O4 each have a variable region). When O1-O4 have a variable region having 5 variable nucleotides, the number of locations to accommodate the possible sequences of the oligos is 4 to the 5^{th} power (4⁵), thus 4x4x4x4x4. Thus, in some embodiments there is a defined oligo sequence at 1024 defined locations, with a single or unique defined sequence present at each location. Thus, O1 oligos can have 1024 possible sequences, which can be present at 1024 defined locations for O1 with a single defined sequence at each location. Each oligo can have more or less than 5 variable nucleotides. It will thus be realized that any of the oligos can have a variable region and/or a degenerate region. In one embodiment only the anchor strands have both a variable region and a degenerate region.

Considering the embodiment of Figure 1A as an example, with oligos 01-06 having five variable nucleotides in a variable region, and five degenerate nucleotides in a degenerate region. In this embodiment O2 and O3 are short oligos, each comprised only of five variable nucleotides; O1 and O4 each have a variable region and will thus each have 1,024 locations. The library can thus have 1024 locations for each of 01-04, i.e. 4,096 locations. O5 and O6 in this example each have five variable nucleotides, and five degenerate nucleotides. Thus, the library can also have 1,024 locations for each of O5 and O6, with each location having a distinct variable sequence, and all possible degenerate sequences, thus 1,024 degenerate oligo sequences are present together at each of the 1,024 locations for each variable sequence. This example thus gives a total of 6,144 distinct locations in the library.

Similarly, considering the embodiment of Figure 1B, oligos O1-O4 can have five variable nucleotides in a variable region. O4 can further have five degenerate nucleotides in a degenerate region, where the degenerate nucleotides can all be co-located with each particular variable sequence. O2 is a short oligo comprised only of five variable nucleotides; O1 and O3 are long oligonucleotides and each have a variable region and will thus each have 1,024 locations. The library can thus have 1,024 locations for each of 01-04, i.e. 4,096 distinct locations in the library. But in other embodiments only some of the oligonucleotides in a binding set can have variable nucleotides.

A location in the oligo library can be a well, a tube, or any other structure that segregates an oligonucleotide member in a distinct location, spatially separated from other members of the library sufficiently for it to be accessed individually and as a species at this distinct location. The oligos can be maintained in their distinct locations as a single molecule (from which a complementary sequence can be synthesized) or as a multiple copies of the same molecule (from which a small volume can be taken and used in synthesis procedures). Some oligonucleotide library members will have more than one actual sequence because of degenerate regions. The distinct locations can be identifiable to a software program that can be configured to a mechanical component or device that retrieves library members from the distinct location for use in a method where the defined oligonucleotide library member is required. In one embodiment an oligo library can be located in a collection of small tubes, each containing a member of the oligo library, and to which instrumentation components can go and retrieve an oligo library member according to instructions located on a non-transitory computer-readable medium. The non-transitory computer readable medium can also contain programmed instructions and/or steps for synthesizing a product DNA molecule, and the programmed instructions and/or steps can be provided to an instrument in communication with the computer-readable medium. The programmed instructions or steps can direct the instrumentation to perform the assembly of a DNA molecule of pre-defined sequence according to any method disclosed herein, or to perform any of the methods provided herein.

Members of the oligonucleotide library are present at distinct locations, spatially separated from other members of the library. A member of the library can be a specific sequence present at its location (either singly or multiple copies). But a member can also be a group of similar sequences present at a distinct or defined location. When degenerate sequences are used, the member of the library containing degenerate sequences can be all possible degenerate sequences (or in some embodiments a subset of all possible sequence) in view of the number of degenerate nucleotides, and present at a distinct location. For example, for an oligonucleotide member having a region of 5 degenerate nucleotides, that member can comprise 4x4x4x4x4 or 1024 distinct oligonucleotides, which can be co-located at a distinct location in the library. Thus, the oligonucleotide library can have fewer than 20,000 distinct locations, with a member of the library being present at the fewer than 20,000 distinct locations. In some embodiments there can be a number of sequences of the all possible sequences that are not of interest. Thus, only a subset of all possible degenerate sequences need be present at the distinct location. Thus, in some embodiments a subset of all possible degenerate sequences can be present as a member of the oligonucleotide library at a distinct location. A distinct location in the oligo library is an identifiable position to which instrumentation can go to obtain the oligonucleotide member at that location. In any embodiment the distinct location can be defined by any suitable technique, for example reference points in a microscopic picture or grid of the solid support containing the oligo library. In some embodiment the distinct location can be stored on and/or communicated by a non-transitory computer-readable medium.

### Product DNA with Overhangs

After removal of flanking sequences (when used), the product DNA molecules can be assembled if desired. In some embodiments the product DNA molecules will be double-stranded blunt end DNA. DNA molecules can be synthesized so that they each contain an overlap or "sticky end" (e.g. one-half the length of the oligo), which can then be used to assemble them into a larger DNA molecule.

But in other embodiments the product DNA molecule can be synthesized having single-stranded overhangs of one or more bases. Type IIS restriction enzymes cleave DNA at a defined distance from their recognition site. As a result Type IIS restriction endonucleases find application in the invention for producing product DNA molecules having single-stranded overhangs. These product DNA molecules can then be joined by annealing to a product DNA molecule having a complementary overhanging sequence to form a larger DNA molecule.

In any embodiment the methods can further involve a step of contacting the product DNA molecule with a Type IIS restriction endonuclease to generate a product DNA molecule having single-stranded overhangs. In any embodiment the methods can also involve a step of joining together two or more of the resultant product DNA molecules to produce a longer product DNA molecule. As shown in Figure 5, recognition sites can be encoded into flanking sequences to enable the release of a double-stranded product DNA molecule having single-stranded overhangs (i.e. "sticky" ends). The single-stranded overhangs can be any suitable length, for example 4 or 5 or 6 or 7 or 8 or more than 8 nucleotides or 6-10 nucleotides or 7-9 nucleotides. In one embodiment the single-stranded overhangs can be one-half the length of the product DNA molecule, or 1 or 2 bases more or less than one-half the length of the product DNA. For example if the length of the product DNA molecule is 16 nucleotides the overhang can be 8 nucleotides or 6-10 nucleotides. In one embodiment BsaI sites can be encoded into the flanking sequences, as depicted in Figure 5. BsaI recognizes the sequence 5'-GGTCTC(N1)-3' (SEQ ID NO: 49). The enzyme generally cleaves to the 3' side of N. Another example of a useful Type IIS restriction enzyme is BsmBI, which recognizes the sequence 5'-CGTCTCN-3' (SEQ ID NO: 50), and generally cleaves to the 3' side of N. Persons of ordinary skill with resort to this disclosure will realize other restriction enzymes that will find use in the invention. Such persons can also easily determine where the enzymes will cut in any particular application. In any embodiment of the methods disclosed herein the DNA molecules produced by the methods can contain one or more Type IIS restriction endonuclease cleavage sites.

### DNA Data Storage

DNA is stable even over periods of thousands of years and even in many extreme environments, giving it great advantages for storing information. Any of the methods disclosed herein can also be applied to encoding digital data into DNA. One or more product DNA molecule(s) can have a sequence that comprises an encoded non-genetic message. One or more product DNA molecule(s) can have a sequence that corresponds to bytes of information that encode the non-genetic message. The bytes of information can be decoded with reference to a key that assigns one or more language character(s) to each encoded character or byte of information. For example, as illustrated in Figure 6, a 16 bp product DNA molecule can be synthesized and easily accommodates four bytes of information, where each byte is encoded by an assigned sequence of nucleotides. In this example a four nucleotide sequence represents a byte of information, which can correspond to a character (e.g. a letter or numeral). Thus, in this example 256 characters can be encoded in each byte of information (4x4x4x4). Thus, the alphabet of any language in the world can be easily accommodated within these 256 bytes of information and a sufficient number of numerals and other characters utilized in communication as well.

The product DNA can also encode a character (e.g. a letter, a word, a number, a punctuation mark, word character, or other characters utilized in communication) indicating where in the sequence the information encoded by that DNA molecule is to be placed. Figure 6 depicts 16 bp product DNA molecules having four bytes of four nucleotides each. The last byte in each product DNA sequence indicates the location in the message where the preceding three bytes are placed; this is conveniently a numeral but can be any character that can be placed into a definable sequence. While a 4 nucleotide byte provides up to 256 identifiers the byte can be any convenient length of nucleotides. For example, bytes can be comprised of 5 nucleotides or 6 nucleotides (allowing for 4,096 identifiers), or 7 or even 8 nucleotides, or more than 8 nucleotides, allowing for many more identifiers to be included. Limited numbers of identifiers can also be expanded by placing DNA molecules in a single well up to the number of identifiers, and then assembling the messages from the DNA in the order of the sequence of wells. Using this method with only a 4 nucleotide identifier even a single 384 well plate can contain over 98,000 DNA molecules (256 molecules x 384 wells), which can be assembled in order to provide almost 300,000 bytes of information (in addition to the identifier). When a 5 nucleotide identifier is used over 1,024 molecules can be individually identified times 384 wells, i.e. 393,000 molecules, or over 1 million bytes of information in a single plate. Multiple plates can be used to accommodate much greater amounts of information. Therefore, an unlimited amount of information can be encoded and stored indefinitely according to the methods.

### CRISPR Guide RNA

The invention can also be applied to the synthesis of guide RNAs (gRNA) for use in CRISPR-Cas9 methods. Using the methods any sequence of gRNA can be quickly constructed. Guide RNA constructs can also be constructed from oligonucleotides in the oligonucleotide library. A product DNA molecule can be synthesized in the methods having a DNA sequence of an initial guide structure. The initial guide RNA structure can encode a gRNA with the necessary prokaryotic or eukaryotic transcriptional elements for in vitro transcription in proper order, for example any one or more of a promoter, a sequence of gRNA, and a terminator. In some embodiments the gRNA can encode a Cas9-binding hairpin (Cas9 handle). In some embodiment the transcriptional elements include a promoter and/or a terminator. In some embodiments the product DNA molecule can encode 20 bases for the gRNA. Figure 7 depicts one embodiment in which short and long oligos of the invention (which can be from an oligo library) are synthesized in the methods into an initial guide structure having the transcriptional elements. Any embodiment of the methods disclosed herein can be used to synthesize the guide structure. Since all possible polynucleotide sequences can be assembled from the oligo library, any initial guide structure can be assembled in the methods. As explained herein product DNA molecules can be synthesized so that they can be ligated to synthesize larger DNA molecules.

### Adding Bases

In any embodiment the methods can include a step that can add 2 or 3 or 4 or 5 or 6 or 7 or 8 or 2-4 or 2-6 or 2-8 or 2-10 or 2-12 extra nucleotides to the product DNA molecule. In the embodiment depicted in Figure 7 a 16 bp product DNA molecule is synthesized by annealing of the short oligonucleotide(s), long oligonucleotides, and anchor strand(s), according to any of the methods disclosed herein. The long and/or short oligos and anchor strand(s) can be made to provide extra nucleotides (e.g. two or more extra nucleotides) that will become part of the product DNA molecule after an amplification step. Any embodiment of the methods can involve multiple steps of PCR or other DNA amplification technique. In any embodiment during a step of PCR primers can be used where the non-standard nucleotides (e.g. dU) are recessed or set back by one or more, or two or more nucleotides from the 3' end of the primers, or set back by 2 or 3 or 4 or 5 or 6 or 7 or 8 or 2-4 or 2-6 or 2-8 or 2-10 or 2-12 nucleotides. This allows for amplification of the extra nucleotide(s) on each side of the product DNA molecule and their inclusion in the product DNA molecule. In some embodiments this is done during a first or early step of PCR. The result is a product DNA molecule that has been extended by, for example, two extra nucleotides on each side. Thus, a 16 bp product DNA molecule can be extended in the method to a 20 bp product DNA molecule.

### Embodiments

Various lengths of long oligonucleotides, short oligonucleotides, and anchor strands can be used in the methods disclosed herein, whether in the embodiment depicted in Figure 1A or the embodiment depicted in Figure 1B. Various sizes of long oligonucleotides, short oligonucleotides, and anchor strands can be used in the methods, and in all possible combinations and sub-combinations.

In some embodiments the at least one long oligonucleotide is from 12-24 nucleotides in length, the at least one short oligonucleotide is 4-8 nucleotides in length, and the at least one anchor strand is from 20-60 nucleotides in length; or the at least one long oligonucleotide is from 12-24 nucleotides in length, the at least one short oligonucleotide is 4-8 nucleotides in length, and the at least one anchor strand is from 12-60 nucleotides in length; or the at least one long oligonucleotide is from 12-24 nucleotides in length, the at least one short oligonucleotide is 4-8 nucleotides in length, and the at least one anchor strand is from 12-80 nucleotides in length. In one embodiment the at least one long oligonucleotide is from 12-24 nucleotides in length, the at least one short oligonucleotide is 4-10 nucleotides in length, and the at least one anchor strand is from 20-60 nucleotides in length; or the at least one long oligonucleotide is from 12-24 nucleotides in length, the at least one short oligonucleotide is 4-10 nucleotides in length, and the at least one anchor strand is from 12-60 nucleotides in length; or the at least one long oligonucleotide is from 12-24 nucleotides in length, the at least one short oligonucleotide is 4-10 nucleotides in length, and the at least one anchor strand is from 12-80 nucleotides in length. In one embodiment the at least one long oligonucleotide is from 12-24 nucleotides in length, the at least one short oligonucleotide is 4-12 nucleotides in length, and the at least one anchor strand is from 20-60 nucleotides in length; or the at least one long oligonucleotide is from 12-24 nucleotides in length, the at least one short oligonucleotide is 4-12 nucleotides in length, and the at least one anchor strand is from 12-60 nucleotides in length; or the at least one long oligonucleotide is from 12-24 nucleotides in length, the at least one short oligonucleotide is 4-12 nucleotides in length, and the at least one anchor strand is from 12-80 nucleotides in length.

In some embodiments the at least one long oligonucleotide is from 12-30 nucleotides in length, and the at least one short oligonucleotide is from 4-8 nucleotides in length, and the at least one anchor strand is from 20-60 nucleotides in length; or the at least one long oligonucleotide is from 12-30 nucleotides in length, and the at least one short oligonucleotide is from either of 4-8 nucleotides in length, and the at least one anchor strand is from 12-60 nucleotides in length; or the at least one long oligonucleotide is from 12-30 nucleotides in length, and the at least one short oligonucleotide is from either of 4-8 nucleotides in length, and the at least one anchor strand is from 12-80 nucleotides in length. In another embodiment the at least one long oligonucleotide is from 12-30 nucleotides in length, and the at least one short oligonucleotide is from 4-10 nucleotides in length, and the at least one anchor strand is from 20-60 nucleotides in length; or the at least one long oligonucleotide is from 12-30 nucleotides in length, and the at least one short oligonucleotide is from either of 4-10 nucleotides in length, and the at least one anchor strand is from 12-60 nucleotides in length; or the at least one long oligonucleotide is from 12-30 nucleotides in length, and the at least one short oligonucleotide is from either of 4-10 nucleotides in length, and the at least one anchor strand is from 12-80 nucleotides in length. In another embodiment the at least one long oligonucleotide is from 12-30 nucleotides in length, and the at least one short oligonucleotide is from 4-12 nucleotides in length, and the at least one anchor strand is from 20-60 nucleotides in length; or the at least one long oligonucleotide is from 12-30 nucleotides in length, and the at least one short oligonucleotide is from either of 4-12 nucleotides in length, and the at least one anchor strand is from 12-60 nucleotides in length; or the at least one long oligonucleotide is from 12-30 nucleotides in length, and the at least one short oligonucleotide is from either of 4-12 nucleotides in length, and the at least one anchor strand is from 12-80 nucleotides in length.

In some embodiments the at least one long oligonucleotide is from 12-40 nucleotides in length, and the at least one short oligonucleotide is from either of 4-8 nucleotides in length, and the at least one anchor strand is from 20-60 nucleotides in length. In another embodiment the at least one long oligonucleotide is from 12-40 nucleotides in length, and the at least one short oligonucleotide is from either of 4-8 nucleotides in length, and the at least one anchor strand is from 12-60 nucleotides in length. In another embodiment the at least one long oligonucleotide is from 12-40 nucleotides in length, and the at least one short oligonucleotide is from either of 4-8 nucleotides in length, and the at least one anchor strand is from 12-80 nucleotides in length. In another embodiment the at least one long oligonucleotide is from 12-40 nucleotides in length, and the at least one short oligonucleotide is from either of 4-10 nucleotides in length, and the at least one anchor strand is from 20-60 nucleotides in length. In another embodiment the at least one long oligonucleotide is from 12-40 nucleotides in length, and the at least one short oligonucleotide is from either of 4-10 nucleotides in length, and the at least one anchor strand is from 12-60 nucleotides in length. In another embodiment the at least one long oligonucleotide is from 12-40 nucleotides in length, and the at least one short oligonucleotide is from either of 4-10 nucleotides in length, and the at least one anchor strand is from 12-80 nucleotides in length. In another embodiment the at least one long oligonucleotide is from 12-40 nucleotides in length, and the at least one short oligonucleotide is from either of 4-12 nucleotides in length, and the at least one anchor strand is from 20-60 nucleotides in length. In another embodiment the at least one long oligonucleotide is from 12-40 nucleotides in length, and the at least one short oligonucleotide is from either of 4-12 nucleotides in length, and the at least one anchor strand is from 12-60 nucleotides in length. In another embodiment the at least one long oligonucleotide is from 12-40 nucleotides in length, and the at least one short oligonucleotide is from either of 4-12 nucleotides in length, and the at least one anchor strand is from 12-80 nucleotides in length.

Furthermore, in any of the embodiments annealing of nucleic acids can begin with denaturation, which can be performed at a temperature of 90 °C or higher, or 95 °C or higher, or 98 °C or higher. Similarly, annealing can occur at temperatures of 35 °C or lower, or 30 °C or lower, or 25 °C or lower. In one embodiment denaturation occurs at 98 °C or higher, and annealing occurs at 25 °C or lower. In another embodiment denaturation occurs at 96 °C or higher, and annealing occurs at 27 °C or lower. The methods can involve transitioning from the denaturation temperature to the annealing temperature at a rate of 1 °C/s or at a rate of 0.1 °C/s. In one embodiment the transition can go from the denaturation temperature to about 85 °C at a rate of 1 °C/s, and then go from about 85 °C to the annealing temperature at a rate of 0.1 °C/s. Annealing and denaturation can be performed in cycles and can be performed in a cycle repeated at least 20 times or at least 25 times or at least 30 times or about 30 times.

Persons of ordinary skill with resort to this disclosure know conditions under which ligation of abutting long and short oligonucleotides can occur while they are bound to the anchor strand(s), which conditions can be readily determined. In various embodiments ligation can occur different temperatures to allow for full settling and annealing of oligonucleotides and anchor strands, and a method can be used to allow for maximal annealing. In various embodiments ligation can occur at for about 5-15 seconds or 8-12 seconds or about 10 seconds at 37 °C ± 2 °C, then about 5-15 seconds or 8-12 seconds or about 10 seconds at 16 °C ± 2 °C, then about 5-15 seconds or 8-12 seconds or about 10 seconds at 4 °C ± 2 °C. In other embodiments ligation can occur at for about 5-15 seconds or 8-12 seconds or about 10 seconds at 37 °C ± 1 °C, then about 5-15 seconds or 8-12 seconds or about 10 seconds at 16 °C ± 1 °C, then about 5-15 seconds or 8-12 seconds or about 10 seconds at 4 °C ± 1 °C.

Persons of ordinary skill with resort to this disclosure also know conditions under which anchor strands containing non-standard nucleotides can be depleted. Conditions can be based on conditions for depleting primers. In various embodiments the conditions can be 35-45 °C for 45-75 minutes, or about 37 °C for 60 minutes followed by 65 °C for 10 minutes.

### Example 1

This examples illustrates synthesis of four different 20mers of varying GC content using an embodiment of the method depicted in Figure 1A. The product DNA molecules (SEQ ID NOs: 1-4) were synthesized with 18 bp flanking sequences. These sequences are provided for illustration and any sequence can be assembled using the methods.

The same procedures were followed for the synthesis of each of SEQ ID NOs: 1-4. For synthesis of SEQ ID NO: 1, mixtures 1 and 2 were formed. Mixture 1 contained restriction enzyme buffer (50 mM potassium acetate, 20 mM Tris-acetate, 10 mM magnesium acetate, and 100 ug/ml BSA, at pH 7.9, commercially available as CutSmart^{®} buffer, New England Biolabs, Inc., Ipswich, MA) to simplify double digest reactions, 100 uM each of O2 (SEQ ID NO: 10), O3 (SEQ ID NO: 11), O5 (SEQ ID NO: 13), and O6 (SEQ ID NO: 14), and water. Mixture 2 contained the same restriction enzyme buffer, 100 uM of O1 (SEQ ID NO: 9) and O4 (SEQ ID NO: 12), and water. The oligonucleotides were as follows:
01, 04 (long oligos): 18 nt (universal flank) + 4-6 nt variable region
O2, O3 (short oligos): 4-6 nt variable region
O5, O6 (anchor strands): 18 nt universal flank + 3-5 nt of degenerate bases + 4-6 nt variable region.

Mixtures 1 and 2 were combined and subjected to denaturation and annealing conditions as follows:
98 °C - 2 min
ramp down to 85 °C at 1 °C/s.
85 °C - 2 min.
ramp down to 25 °C at 0.1 °C/s
25 °C - 2 min.

After denaturation and annealing the mixture was subjected to the ligation reaction, which was conducted with T4 ligase under a ligation protocol, which included 30 cycles of:
37°C - 10 s
16 °C - 10 s
4 °C - 10 s

The same cycles were then repeated 30 more times to end with 70 °C for 15 min in preparation for anchor strand depletion.

The mixture was then subjected to an anchor strand depletion step using uracil DNA glycosylase (UDG) and DNA glycosylase-lyase endonuclease VIII for uracil-specific excision that generates a single nucleotide gap at the location of a uracil residue. The reaction conditions were 37 °C for 60 minutes followed by 65 °C for 10 minutes.

The anchor strand depletion mixture was diluted 1:100 and 2 ul was used as a template in the final PCR amplification reaction. The PCR reaction components included the DNA polymerase (VeraSeq^{®} 2.0 high-fidelity DNA polymerase (Qiagen Beverly, LLC, Beverly, MA,), universal primers, dNTPs, and template. The PCR protocol was as follows:
98 °C - 30 s
30 cycles of
   98 °C - 10s
   60 °C - 10s
   72 °C - 15 s
72 °C - 10 min

As show in Figure 3, all four product DNA molecules of 20 bp and of varied GC content were assembled with 18 bp flanking sequences to produce 56 bp amplicons, which were resolved on a 4% agarose gel. SEQ ID NO: 1-4 were confirmed by Sanger sequencing of 24 clones and found to have the expected sequence.

Synthesis of SEQ ID NOs: 2-4 was the same, using the following oligonucleotides from the oligo library.

For SEQ ID NO: 2 - 01 (SEQ ID NO: 15), 02 (SEQ ID NO: 16), O3 (SEQ ID NO: 17), 04 (SEQ ID NO: 18), O5 (SEQ ID NO: 19), 06 (SEQ ID NO: 20).

For SEQ ID NO: 3 - O1 (SEQ ID NO: 21), O2 (SEQ ID NO: 22), O3 (SEQ ID NO: 23), O4 (SEQ ID NO: 24), O5 (SEQ ID NO: 25), O6 (SEQ ID NO: 26).

For SEQ ID NO: 4 - O1 (SEQ ID NO: 27), O2 (SEQ ID NO: 28), O3 (SEQ ID NO: 29), O4 (SEQ ID NO: 30), O5 (SEQ ID NO: 31), O6 (SEQ ID NO: 32).

As illustrated in Figure 3 and confirmed by Sanger sequencing, all sequences were shown to have the correct and defined sequence.

### Example 2

This example shows another embodiment illustrating synthesis of four product DNA molecules (SEQ ID NOs: 5-8) where the binding sets contained two long oligonucleotides, one short oligonucleotide, and one anchor strand, an embodiment of which is depicted in Figure 1B. The 20 bp product DNA molecules were synthesized with 18 bp flanking sequences, providing a final amplicon length of 56 bp.

The same procedures were followed for the synthesis of each of SEQ ID NOs: 5-8. For synthesis of SEQ ID NO: 5, mixtures O1 (SEQ ID NO: 33), O2 (SEQ ID NO: 34), O3 (SEQ ID NO: 35) and O4 (SEQ ID NO: 36) were contained in a mixture. O1 and O3 are long oligonucleotides, O2 is a short oligonucleotide, and O4 is the anchor strand. The oligonucleotides were as follows:
O1, O3 (long oligos): 18 nt universal flanks + 4-6 nt variable region
O2 (short oligo): 6 nt variable region
O4 (anchor strand): 52 nt

Protocols were the same as those in Example 1. The result is shown in Figure 4, which shows an agarose gel with the four amplicons resolved. The four 16 bp DNA products (SEQ ID NOs: 5-8) were successfully assembled with their 18 bp flanking sequences to result in 52 bp amplicons. Assembly was demonstrated on a 4% agarose gel (Figure 3). The sequences of the final products were confirmed by Sanger sequencing and found to be the expected sequences.

Synthesis of SEQ ID NOs: 6-8 were performed in the same manner, using the following oligonucleotides from the oligo library.

For SEQ ID NO: 6 - O1 (SEQ ID NO: 37), O2 (SEQ ID NO: 38), O3 (SEQ ID NO: 39), O4 (SEQ ID NO: 40);

For SEQ ID NO: 7 - O1 (SEQ ID NO: 41), O2 (SEQ ID NO: 42), O3 (SEQ ID NO: 43), O4 (SEQ ID NO: 44);

For SEQ ID NO: 8 - O1 (SEQ ID NO: 45), O2 (SEQ ID NO: 46), O3 (SEQ ID NO: 47), O4 (SEQ ID NO: 48);

As illustrated in Figure 4 (and confirmed by Sanger sequencing), all sequences were shown to have the correct and defined sequence.

## Claims

1. A method of synthesizing a product DNA molecule having a desired sequence comprising:
annealing at least one long oligonucleotide and at least one short oligonucleotide to at least one anchor strand(s) having a sequence at least partially complementary to the at least one long and at least one short oligonucleotide,
wherein after annealing, at least one long oligonucleotide bound to an anchor strand abuts at least one short oligonucleotide bound to the same anchor strand, and
wherein the at least one anchor strand comprises one or more non-standard nucleotides, and one or more degenerate nucleotides;
ligating the abutting at least one long oligonucleotide and at least one short oligonucleotide to form a dsDNA molecule comprising non-standard nucleotides;
contacting the dsDNA molecule with one or more enzymes that degrade DNA comprising one or more non-standard nucleotides to thereby synthesize the product DNA molecule.

2. The method of claim 1 wherein the annealing occurs within a binding set comprising two long oligonucleotides, two short oligonucleotides, and two anchor strands, and further comprising ligating the two short oligonucleotides to the two long oligonucleotides, wherein the long and short oligonucleotides further comprise variable nucleotides.

3. The method of claim 1 wherein the annealing occurs within a binding set comprising two long oligonucleotides, one short oligonucleotide, and at least one anchor strand, and wherein the short oligonucleotide is ligated to two long oligonucleotides, and the short oligonucleotide and two long oligonucleotides comprise variable nucleotides.

4. The method of any one of claims 1-3 wherein (i) the non-standard nucleotide is deoxy-uridine; and/or (ii) the dsDNA molecule comprises the non-standard nucleotides on only one strand.

5. The method of any one of claims 1-4 further comprising amplifying the product DNA molecule using a DNA amplification method.

6. The method of claim 5 wherein the amplifying is performed by the polymerase chain reaction (PCR) or wherein the amplifying comprises performing multiple cycles of PCR on the product DNA molecule.

7. The method of any one of claims 1-6 (i) wherein the product DNA molecule comprises flanking sequences for annealing a primer used in the multiple cycles of PCR; optionally wherein the product DNA molecule encodes a guide RNA, and the flanking sequences encode one or more transcriptional elements, and the guide RNA optionally encodes a Cas9-binding hairpin; optionally wherein the transcriptional elements comprise a promoter and a terminator; (ii) wherein the product DNA molecule is 8-30 nucleotides in length; (iii) wherein the ligation is performed by a DNA ligase; (iv) wherein the at least one long oligonucleotide is from 12-24 nucleotides in length, and the at least one short oligonucleotide is from 4-8 nucleotides in length; optionally wherein the at least one anchor strand is from 20-60 nucleotides in length; (v) further comprising ligating in solution two DNA molecules having double-stranded ends by blunt end ligation and according to the desired sequence; (vi) wherein the one or more enzymes that degrade DNA comprising non-standard nucleotides comprise one or more enzymes selected from the group consisting of: uracil DNA glycosylase, Endonuclease VIII, and Exonuclease T; and/or (vii) wherein each anchor strand comprises 4-6 variable and/or 4-6 degenerate nucleotides comprised in a single degenerate region.

8. The method of any one of claims 2 or 4-7 further comprising that after annealing, a first short oligonucleotide abuts a first long oligonucleotide, and a second short oligonucleotide abuts a second long oligonucleotide, and ligating the first short and long oligonucleotides, and ligating the second short and long oligonucleotides.

9. The method of any one of claims 3-7 further comprising that after annealing the at least one short oligonucleotide abuts a first long oligonucleotide and a second long oligonucleotide, and ligating the at least one short oligonucleotide to the first and second long oligonucleotides.

10. The method of claim 3 wherein the anchor strand comprises 4-6 variable and/or 4-6 degenerate nucleotides comprised in a single degenerate region.

11. The method of any one of claims 1-10 wherein (i) the long and short oligonucleotides each comprise 4-6 variable nucleotides; (ii) the two long oligonucleotides further comprise variable nucleotides that bind to the degenerate nucleotides on their respective at least partially complementary anchor strands during the annealing step; (iii) the long oligonucleotides, short oligonucleotides, and anchor strand each comprise 4-6 variable nucleotides; and/or (iv) the product DNA molecule is single-stranded DNA 8-30 nucleotides in length, not including the flanking sequences; or wherein the product DNA molecule is double-stranded DNA 8-30 base pairs in length, not including the flanking sequences; or (v) the product DNA molecule is single-stranded DNA 16-20 oligonucleotide in length; or wherein the product DNA molecule is double-stranded DNA 16-20 base pairs in length; and/or (vi) the long oligonucleotides and short oligonucleotides are provided to the method from an oligonucleotide library; optionally wherein the short oligonucleotides provided from the oligonucleotide library are 3-7 nucleotides in length; optionally wherein the oligonucleotide library comprises long oligonucleotide members having all four possible nucleotides at each position of a variable nucleotide; and/or (vii) the long oligonucleotides comprise a variable region that anneals to the sequence of degenerate nucleotides on the one or more anchor strands.

12. The method of any one of claims 2 or 4-11 wherein
a. the short and long oligos comprise 4-6 variable nucleotides;
b. the anchor strands comprise 4-6 degenerate nucleotides;
c. the anchor strands comprise at least two deoxy-uridine monophosphate nucleotides; and
d. the product DNA molecule is amplified by PCR.

13. The method of any one of claims 3-11 wherein
a. the short and long oligos comprise 4-6 variable nucleotides;
b. the anchor strand comprises 4-6 degenerate nucleotides;
c. the anchor strand comprises at least two deoxy-uracil monophosphate nucleotides; and
d. the product DNA molecule is amplified by PCR.

14. The method of any one of claims 1-13 (i) wherein the product DNA molecule has an error rate of fewer than 1 in 25,000; (ii) wherein the at least one long oligonucleotide, at least one short oligonucleotide, and at least one anchor strand are selected from an oligonucleotide library comprising less than 20,000 members; optionally wherein the oligonucleotide library comprising less than 20,000 members contains oligonucleotides sufficient to synthesize all possible nucleotide sequences; (iii) further comprising a step of contacting the product DNA molecule with a Type IIS restriction endonuclease to generate a product DNA molecule having single-stranded overhangs; optionally wherein in the Type IIS restriction endonuclease is selected from BsaI and BsmBI; (iv) wherein the product DNA molecule comprises an encoded non-genetic message; and/or (iv) wherein the product DNA molecule comprises a sequence corresponding to bytes of information.

15. The method of claim 6 wherein the multiple cycles of PCR comprise a first step of PCR using primers comprising non-standard nucleotides set two nucleotides back from the 3' end of the primer.

16. A method of synthesizing a product DNA molecule comprising:
providing a library of oligonucleotides wherein the library of oligonucleotides comprises fewer than 20,000 oligonucleotide members, and wherein the oligonucleotide members in the library can be assembled into all possible polynucleotide sequences;
assembling oligonucleotide members from the library utilizing a method of any one of claims 1-15 to obtain the product DNA molecule; optionally wherein (i) the library of oligonucleotides is present on a solid phase and the oligonucleotide members are present at defined physical locations within the library of oligonucleotides; optionally wherein the solid phase is a DNA chip; (ii) the product DNA molecule is double-stranded DNA 8-30 base pairs in length; (iii) the product DNA molecule is subjected to continuing methods to ligate the product DNA molecule with at least one additional DNA molecule to produce a larger product DNA molecule; (iv) the library comprises fewer than 16,000 oligonucleotide members; (v) the larger product DNA molecule is at least 1,000 bp in length; and/or (vi) the product DNA molecule has an error rate of fewer than 1 in 10,000.

## Patentansprüche

1. Verfahren zum Synthetisieren eines Produkt-DNA-Moleküls mit einer gewünschten Sequenz, umfassend:
Annealing wenigstens eines langen Oligonukleotids und wenigstens eines kurzen Oligonukleotids an wenigstens einen Ankerstrang mit einer Sequenz, die wenigstens teilweise komplementär zu dem wenigstens einen langen und wenigstens einen kurzen Oligonukleotid ist,
wobei nach Annealing wenigstens ein langes Oligonukleotid, das an einen Ankerstrang gebunden ist, an wenigstens ein kurzes Oligonukleotid, das an den gleichen Ankerstrang gebunden ist, angrenzt, und
wobei der wenigstens eine Ankerstrang ein oder mehrere Nicht-Standard-Nukleotide und ein oder mehrere degenerierte Nukleotide umfasst;
Ligieren des angrenzenden wenigstens einen langen Oligonukleotids und wenigstens eines kurzen Oligonukleotids, um ein dsDNA-Molekül zu bilden, das Nicht-Standard-Nukleotide umfasst;
Inkontaktbringen des dsDNA-Moleküls mit einem oder mehreren Enzymen, die DNA abbauen, die ein oder mehrere Nicht-Standard-Nukleotide umfasst, um dadurch das Produkt-DNA-Molekül zu synthetisieren.

2. Verfahren nach Anspruch 1, wobei das Annealing innerhalb eines Bindungssets erfolgt, das zwei lange Oligonukleotide, zwei kurze Oligonukleotide und zwei Ankerstränge umfasst, und ferner umfassend Ligieren der beiden kurzen Oligonukleotide an die beiden langen Oligonukleotide, wobei die langen und kurzen Oligonukleotide ferner variable Nukleotide umfassen.

3. Verfahren nach Anspruch 1, wobei das Annealing innerhalb eines Bindungssets erfolgt, das zwei lange Oligonukleotide, ein kurzes Oligonukleotid und wenigstens einen Ankerstrang umfasst, und wobei das kurze Oligonukleotid an zwei lange Oligonukleotide ligiert wird und das kurze Oligonukleotid und die beiden langen Oligonukleotide variable Nukleotide umfassen.

4. Verfahren nach einem der Ansprüche 1-3, wobei (i) das Nicht-Standard-Nukleotid Desoxyuridin ist; und/oder (ii) das dsDNA-Molekül die Nicht-Standard-Nukleotide auf nur einem Strang umfasst.

5. Verfahren nach einem der Ansprüche 1-4, ferner umfassend Amplifizieren des Produkt-DNA-Moleküls unter Verwendung eines DNA-Amplifikationsverfahrens.

6. Verfahren nach Anspruch 5, wobei das Amplifizieren durch die Polymerasekettenreaktion (PCR) durchgeführt wird oder wobei das Amplifizieren Durchführen mehrerer PCR-Zyklen an dem Produkt-DNA-Molekül umfasst.

7. Verfahren nach einem der Ansprüche 1-6, (i) wobei das Produkt-DNA-Molekül flankierende Sequenzen zum Annealing eines Primers umfasst, der in den mehreren PCR-Zyklen verwendet wird; gegebenenfalls wobei das Produkt-DNA-Molekül eine Guide-RNA codiert und die flankierenden Sequenzen ein oder mehrere Transkriptionselemente codieren und die Guide-RNA gegebenenfalls eine Cas9-bindende Haarnadel codiert; gegebenenfalls wobei die Transkriptionselemente einen Promotor und einen Terminator umfassen; (ii) wobei das Produkt-DNA-Molekül 8-30 Nukleotide lang ist; (iii) wobei die Ligation durch eine DNA-Ligase durchgeführt wird; (iv) wobei das wenigstens eine lange Oligonukleotid 12-24 Nukleotide lang ist und das wenigstens eine kurze Oligonukleotid 4-8 Nukleotide lang ist; gegebenenfalls wobei der wenigstens eine Ankerstrang 20-60 Nukleotide lang ist; (v) ferner umfassend Ligieren in Lösung von zwei DNA-Molekülen mit doppelsträngigen Enden durch Blunt-End-Ligation und gemäß der gewünschten Sequenz; (vi) wobei das eine oder die mehreren Enzyme, die DNA abbauen, die Nicht-Standard-Nukleotide umfasst, ein oder mehrere Enzyme umfassen, die ausgewählt sind aus der Gruppe bestehend aus: Uracil-DNA-Glycosylase, Endonuklease VIII und Exonuklease T; und/oder (vii) wobei jeder Ankerstrang 4-6 variable und/oder 4-6 degenerierte Nukleotide umfasst, die in einer einzelnen degenerierten Region enthalten sind.

8. Verfahren nach einem der Ansprüche 2 oder 4-7, ferner umfassend, dass nach Annealing ein erstes kurzes Oligonukleotid an ein erstes langes Oligonukleotid angrenzt und ein zweites kurzes Oligonukleotid an ein zweites langes Oligonukleotid angrenzt, und Ligieren der ersten kurzen und langen Oligonukleotide und Ligieren der zweiten kurzen und langen Oligonukleotide.

9. Verfahren nach einem der Ansprüche 3-7, ferner umfassend, dass nach Annealing das wenigstens eine kurze Oligonukleotid an ein erstes langes Oligonukleotid und ein zweites langes Oligonukleotid angrenzt und das wenigstens eine kurze Oligonukleotid an das erste und das zweite lange Oligonukleotid ligiert wird.

10. Verfahren nach Anspruch 3, wobei der Ankerstrang 4-6 variable und/oder 4-6 degenerierte Nukleotide umfasst, die in einer einzelnen degenerierten Region enthalten sind.

11. Verfahren nach einem der Ansprüche 1-10, wobei (i) die langen und kurzen Oligonukleotide jeweils 4-6 variable Nukleotide umfassen; (ii) die beiden langen Oligonukleotide ferner variable Nukleotide umfassen, die an die degenerierten Nukleotide an ihren entsprechenden wenigstens teilweise komplementären Ankersträngen während des Annealing-Schritts binden; (iii) die langen Oligonukleotide, kurzen Oligonukleotide und der Ankerstrang jeweils 4-6 variable Nukleotide umfassen; und/oder (iv) das Produkt-DNA-Molekül 8-30 Nukleotide lange einzelsträngige DNA ist, nicht einschließlich der flankierenden Sequenzen; oder wobei das Produkt-DNA-Molekül 8-30 Nukleotide lange doppelsträngige DNA ist, nicht einschließlich der flankierenden Sequenzen; oder (v) das Produkt-DNA-Molekül 16-20 Oligonukleotide lange einzelsträngige DNA ist; oder wobei das Produkt-DNA-Molekül 16-20 Basenpaare lange doppelsträngige DNA ist; und/oder (vi) die langen Oligonukleotide und kurzen Oligonukleotide dem Verfahren aus einer Oligonukleotidbibliothek bereitgestellt werden; gegebenenfalls wobei die kurzen Oligonukleotide, aus der Oligonukleotidbibliothek bereitgestellt werden, 3-7 Nukleotide lang sind; wobei gegebenenfalls die Oligonukleotidbibliothek lange Oligonukleotidelemente mit allen vier möglichen Nukleotiden an jeder Position eines variablen Nukleotids umfasst; und/oder (vii) die langen Oligonukleotide eine variable Region umfassen, die an die Sequenz von degenerierten Nukleotiden an dem einen oder den mehreren Ankersträngen anlagert.

12. Verfahren nach einem der Ansprüche 2 oder 4-11, wobei:
a. die kurzen und langen Oligos 4-6 variable Nukleotide umfassen;
b. die Ankerstränge 4-6 degenerierte Nukleotide umfassen;
c. die Ankerstränge wenigstens zwei Desoxyuridinmonophosphat-Nukleotide umfassen; und
d. das Produkt-DNA-Molekül durch PCR amplifiziert wird.

13. Verfahren nach einem der Ansprüche 3-11, wobei
a. die kurzen und langen Oligos 4-6 variable Nukleotide umfassen;
b. der Ankerstrang 4-6 degenerierte Nukleotide umfasst;
c. der Ankerstrang wenigstens zwei Desoxyuracilmonophosphat-Nukleotide umfasst; und
d. das Produkt-DNA-Molekül durch PCR amplifiziert wird.

14. Verfahren nach einem der Ansprüche 1-13, (i) wobei das Produkt-DNA-Molekül eine Fehlerrate von weniger als 1 in 25.000 aufweist; (ii) wobei das wenigstens eine lange Oligonukleotid, wenigstens ein kurzes Oligonukleotid und wenigstens ein Ankerstrang aus einer Oligonukleotidbibliothek ausgewählt werden, die weniger als 20.000 Mitglieder umfasst; wobei gegebenenfalls die Oligonukleotidbibliothek, die weniger als 20.000 Mitglieder umfasst, Oligonukleotide enthält, die ausreichen, um alle möglichen Nukleotidsequenzen zu synthetisieren; (iii) ferner umfassend einen Schritt des Inkontaktbringens des Produkt-DNA-Moleküls mit einer Typ-IIS-Restriktionsendonuklease, um ein Produkt-DNA-Molekül mit einzelsträngigen Überhängen zu erzeugen; gegebenenfalls wobei die Typ-IIS-Restriktionsendonuklease ausgewählt ist aus BsaI und BsmBI; (iv) wobei das Produkt-DNA-Molekül eine codierte nicht-genetische Nachricht umfasst; und/oder (iv) wobei das Produkt-DNA-Molekül eine Sequenz umfasst, die Informationsbytes entspricht.

15. Verfahren nach Anspruch 6, wobei die mehreren PCR-Zyklen einen ersten Schritt von PCR unter Verwendung von Primern umfassen, die Nicht-Standard-Nukleotide umfassen, die zwei Nukleotide von dem 3'-Ende des Primers zurückgesetzt sind.

16. Verfahren zum Synthetisieren eines Produkt-DNA-Moleküls, umfassend:
Bereitstellen einer Bibliothek von Oligonukleotiden, wobei die Bibliothek von Oligonukleotiden weniger als 20.000 Oligonukleotidelemente umfasst und wobei die Oligonukleotidmitelemente in der Bibliothek zu allen möglichen Polynukleotidsequenzen zusammengesetzt werden können;
Zusammensetzen von Oligonukleotidelementen aus der Bibliothek unter Verwendung eines Verfahrens nach einem der Ansprüche 1-15, um das Produkt-DNA-Molekül zu erhalten; gegebenenfalls wobei (i) die Bibliothek von Oligonukleotiden auf einer festen Phase vorliegt und die Oligonukleotidelemente an definierten physischen Orten innerhalb der Bibliothek von Oligonukleotiden vorliegen;
gegebenenfalls wobei die feste Phase ein DNA-Chip ist; (ii) das Produkt-DNA-Molekül 8-30 Basenpaare lange doppelsträngige DNA ist; (iii) das Produkt-DNA-Molekül fortgesetzten Verfahren unterzogen wird, um das Produkt-DNA-Molekül mit wenigstens einem zusätzlichen DNA-Molekül zu ligieren, um ein größeres Produkt-DNA-Molekül zu erzeugen; (iv) die Bibliothek weniger als 16.000 Oligonukleotidelemente umfasst; (v) das größere Produkt-DNA-Molekül wenigstens 1.000 bp lang ist; und/oder (vi) das Produkt-DNA-Molekül eine Fehlerrate von weniger als 1 in 10.000 aufweist.

## Revendications

1. Procédé de synthèse d'une molécule d'ADN produit ayant une séquence souhaitée comprenant :
l'hybridation d'au moins un oligonucléotide long et d'au moins un oligonucléotide court en au moins un brin d'ancrage ayant une séquence au moins partiellement complémentaire du ou des oligonucléotides longs et du ou des oligonucléotides courts,
dans lequel, après l'hybridation, au moins un oligonucléotide long lié à un brin d'ancrage est adjacent à au moins un oligonucléotide court lié au même brin d'ancrage, et
dans lequel le ou les brins d'ancrage comprennent un ou plusieurs nucléotides non standard, et un ou plusieurs nucléotides dégénérés ;
la ligature du ou des oligonucléotides longs et du ou des oligonucléotides courts adjacents pour former une molécule d'ADNdb comprenant des nucléotides non standard ;
la mise en contact de la molécule d'ADNdb avec une ou plusieurs enzymes qui dégradent l'ADN comprenant un ou plusieurs nucléotides non standard afin de synthétiser ainsi la molécule d'ADN produit.

2. Procédé selon la revendication 1, dans lequel l'hybridation se produit dans un ensemble de liaison comprenant deux oligonucléotides longs, deux oligonucléotides courts et deux brins d'ancrage, et comprenant en outre la ligature des deux oligonucléotides courts aux deux oligonucléotides longs, les oligonucléotides long et court comprenant en outre des nucléotides variables.

3. Procédé selon la revendication 1, dans lequel l'hybridation se produit dans un ensemble de liaison comprenant deux oligonucléotides longs, un oligonucléotide court et au moins un brin d'ancrage, et dans lequel l'oligonucléotide court est ligaturé à deux oligonucléotides longs, et l'oligonucléotide court et deux oligonucléotides longs comprennent des nucléotides variables.

4. Procédé selon l'une quelconque des revendications 1 à 3 dans lequel (i) le nucléotide non standard est la désoxyuridine ; et/ou (ii) la molécule d'ADNdb comprend les nucléotides non standard sur un seul brin.

5. Procédé selon l'une quelconque des revendications 1 à 4 comprenant en outre l'amplification de la molécule d'ADN produit selon un procédé d'amplification d'ADN.

6. Procédé selon la revendication 5, dans lequel l'amplification est réalisée par réaction en chaîne par polymérase (PCR) ou dans lequel l'amplification comprend la réalisation de cycles multiples de PCR sur la molécule d'ADN produit.

7. Procédé selon l'une quelconque des revendications 1 à 6 (i) dans lequel la molécule d'ADN produit comprend des séquences flanquantes pour hybrider une amorce utilisée dans les cycles multiples de PCR ; facultativement, dans lequel la molécule d'ADN produit code pour un ARN guide, et les séquences flanquantes codent pour un ou plusieurs éléments transcriptionnels, et l'ARN guide code facultativement pour une épingle à cheveux se liant à Cas9 ; facultativement, dans lequel les éléments transcriptionnels comprennent un promoteur et un terminateur ; (ii) dans lequel la molécule d'ADN produit a une longueur de 8 à 30 nucléotides ; (iii) dans lequel la ligature est effectuée par une ADN ligase ; (iv) dans lequel le ou les oligonucléotides longs ont une longueur de 12 à 24 nucléotides et le ou les oligonucléotides courts ont une longueur de 4 à 8 nucléotides ; facultativement, dans lequel le ou les brins d'ancrage ont une longueur de 20 à 60 nucléotides ; (v) comprenant en outre la ligature en solution de deux molécules d'ADN ayant des extrémités double brin par ligature à extrémités franches et conformément à la séquence souhaitée ; (vi) dans lequel les une ou plusieurs enzymes qui dégradent l'ADN comprenant des nucléotides non standard comprennent une ou plusieurs enzymes choisies dans le groupe constitué par : l'uracile ADN glycosylase, l'endonucléase VIII et l'exonucléase T ; et/ou (vii) dans lequel chaque brin d'ancrage comprend 4 à 6 nucléotides variables et/ou 4 à 6 nucléotides dégénérés compris dans une seule région dégénérée.

8. Procédé selon l'une quelconque des revendications 2 ou 4 à 7, dans lequel, en outre, après l'hybridation, un premier oligonucléotide court est adjacent à un premier oligonucléotide long, et un deuxième oligonucléotide court est adjacent à un deuxième oligonucléotide long, et comprenant la ligature des premier oligonucléotides court et long, et la ligature des deuxièmes oligonucléotides court et long.

9. Procédé selon l'une quelconque des revendications 3 à 7 dans lequel, après l'hybridation, le ou les oligonucléotides courts sont adjacents à un premier oligonucléotide long et à un deuxième oligonucléotide long, et comprenant la ligature du ou des oligonucléotides courts aux premier et deuxième oligonucléotides longs.

10. Procédé selon la revendication 3, dans lequel le brin d'ancrage comprend 4 à 6 nucléotides variables et/ou 4 à 6 nucléotides dégénérés compris dans une seule région dégénérée.

11. Procédé selon l'une quelconque des revendications 1 à 10 dans lequel (i) les oligonucléotides longs et courts comprennent chacun 4 à 6 nucléotides variables ; (ii) les deux oligonucléotides longs comprennent en outre des nucléotides variables qui se lient aux nucléotides dégénérés sur leurs brins d'ancrage au moins partiellement complémentaires respectifs pendant l'étape d'hybridation ; (iii) les oligonucléotides longs, les oligonucléotides courts et le brin d'ancrage comprennent chacun 4 à 6 nucléotides variables ; et/ou (iv) la molécule d'ADN produit est un ADN simple brin ayant une longueur de 8 à 30 nucléotides, séquences flanquantes non incluses ; ou dans lequel la molécule d'ADN produit est un ADN double brin ayant une longueur de 8 à 30 paires de bases, séquences flanquantes non incluses ; ou (v) la molécule d'ADN produit est un ADN simple brin ayant une longueur de 16 à 20 nucléotides ; ou dans lequel la molécule d'ADN produit est un ADN double brin ayant une longueur de 16 à 20 paires de bases ; et/ou (vi) les oligonucléotides longs et les oligonucléotides courts utilisés dans le procédé proviennent d'une bibliothèque d'oligonucléotides ; facultativement, dans lequel les oligonucléotides courts provenant d'une bibliothèque d'oligonucléotides ont une longueur de 3 à 7 nucléotides ; facultativement, dans lequel la bibliothèque d'oligonucléotides comprend des oligonucléotides longs contenant les quatre nucléotides possibles à chaque position d'un nucléotide variable ; et/ou (vii) les oligonucléotides longs comprennent une région variable qui s'hybride à la séquence de nucléotides dégénérés sur le ou les brins d'ancrage.

12. Procédé selon l'une quelconque des revendications 2 ou 4 à 11, dans lequel
a. les oligonucléotides courts et longs comprennent 4 à 6 nucléotides variables ;
b. les brins d'ancrage comprennent 4 à 6 nucléotides dégénérés ;
c. les brins d'ancrage comprennent au moins deux nucléotides de désoxyuridine monophosphate ; et
d. la molécule d'ADN produit est amplifiée par PCR.

13. Procédé selon l'une quelconque des revendications 3 à 11, dans lequel
a. les oligonucléotides courts et longs comprennent 4 à 6 nucléotides variables ;
b. le brin d'ancrage comprend 4 à 6 nucléotides dégénérés ;
c. le brin d'ancrage comprend au moins deux nucléotides de désoxyuracile monophosphate ; et
d. la molécule d'ADN produit est amplifiée par PCR.

14. Procédé selon l'une quelconque des revendications 1 à 13 (i) dans lequel la molécule d'ADN produit présente un taux d'erreur inférieur à 1 sur 25 000 ; (ii) dans lequel le ou les oligonucléotides longs, le ou les oligonucléotides courts et le ou les brins d'ancrage sont choisis dans une bibliothèque d'oligonucléotides comprenant moins de 20 000 membres ; facultativement, dans lequel la bibliothèque d'oligonucléotides comprenant moins de 20 000 membres contient suffisamment d'oligonucléotides pour synthétiser toutes les séquences nucléotidiques possibles ; (iii) comprenant en outre une étape de mise en contact de la molécule d'ADN produit avec une endonucléase de restriction de type IIS pour générer une molécule d'ADN produit ayant des extrémités saillantes simple brin ; facultativement, dans lequel l'endonucléase de restriction de type IIS est choisie parmi BsaI et BsmBI ; (iv) dans laquelle la molécule d'ADN produit comprend un message non génétique codé ; et/ou (iv) dans lequel la molécule d'ADN produit comprend une séquence correspondant à des octets d'informations.

15. Procédé selon la revendication 6, dans lequel les cycles multiples de PCR comprennent une première étape de PCR utilisant des amorces comprenant des nucléotides non standard situés à deux nucléotides de l'extrémité 3' de l'amorce.

16. Procédé de synthèse d'une molécule d'ADN produit comprenant :
la fourniture d'une bibliothèque d'oligonucléotides, la bibliothèque d'oligonucléotides comprenant moins de 20 000 oligonucléotides, et les oligonucléotides de la bibliothèque peuvent être assemblés selon toutes les séquences polynucléotidiques possibles ;
l'assemblage d'oligonucléotides provenant de la bibliothèque au moyen d'un procédé selon l'une quelconque des revendications 1 à 15 pour obtenir la molécule d'ADN produit ; facultativement dans lequel (i) la bibliothèque d'oligonucléotides est présente sur une phase solide et les oligonucléotides sont présents à des emplacements physiques définis dans la bibliothèque d'oligonucléotides ; facultativement dans lequel la phase solide est une puce à ADN ; (ii) la molécule d'ADN produit est un ADN double brin ayant une longueur de 8 à 30 paires de bases ; (iii) la molécule d'ADN produit est soumise à des procédés continus pour ligaturer la molécule d'ADN produit avec au moins une molécule d'ADN supplémentaire afin de produire une molécule d'ADN produit plus grande ; (iv) la bibliothèque comprend moins de 16 000 oligonucléotides ; (v) la molécule d'ADN produit plus grande a une longueur d'au moins 1 000 pb ;
et/ou (vi) la molécule d'ADN produit présente un taux d'erreur inférieur à 1 sur 10 000.
